(19) **Europäisches Patentamt**
**European Patent Office**
**Office européen des brevets**

(11) **EP 1 636 727 B1**

(12) **EUROPEAN PATENT SPECIFICATION**

(45) Date of publication and mention
of the grant of the patent:
**31.10.2012 Bulletin 2012/44**

(51) Int Cl.:
**G06F 19/18** (2011.01)

(21) Application number: **04741774.6**

(86) International application number:
**PCT/EP2004/051084**

(22) Date of filing: **10.06.2004**

(87) International publication number:
**WO 2004/111907 (23.12.2004 Gazette 2004/52)**

(54) **COMPUTATIONAL METHOD FOR PREDICTING THE CONTRIBUTION OF MUTATIONS TO THE DRUG RESISTANCE PHENOTYPE EXHIBITED BY HIV BASED ON A LINEAR REGRESSION ANALYSIS OF THE LOG FOLD RESISTANCE**

RECHNERISCHES VERFAHREN ZUR VORHERSAGE DES BEITRAGS VON MUTATIONEN ZU DEM DURCH HIV GEZEIGTEN MEDIKAMENTENRESISTENZPHÄNOTYP AUF DER BASIS EINER LINEAREN REGRESSIONSANALYSE DER LOG-FALTRESISTENZ

PROCEDE INFORMATIQUE DE PREVISION DE LA CONTRIBUTION DES MUTATIONS A LA RESISTANCE DE VIH AUX MEDICAMENTS BASE SUR L'ANALYSE DE REGRESSION LINEAIRE DU LOGARITHME DE LA RESISTANCE DE PLIAGE

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **10.06.2003 EP 03101687**
**16.06.2003 US 478780 P**

(43) Date of publication of application:
**22.03.2006 Bulletin 2006/12**

(73) Proprietor: **Janssen Diagnostics BVBA**
**2340 Beerse (BE)**

(72) Inventors:
- **VAN MARCK, Herwig, Gaston, Emiel**
**B-9810 Nazareth (BE)**
- **VAN DEN BULCKE, Tim, Gerrit**
**B-2800 Mechelen (BE)**
- **VERMEIREN, Hans, Victor**
**B-2812 Muizen (BE)**

(74) Representative: **van Wanrooij, Eva et al**
**J&J Patent Law Department**
**Turnhoutseweg 30**
**2340 Beerse (BE)**

(56) References cited:
**WO-A-01/79540**

- **BEERENWINKEL NIKO ET AL: "Diversity and complexity of HIV-1 drug resistance: A bioinformatics approach to predicting phenotype from genotype" PROCEEDINGS OF THE NATIONAL ACADEMY OF SCIENCES OF THE UNITED STATES, vol. 99, no. 12, 11 June 2002 (2002-06-11), pages 8271-8276, XP002259243 June 11, 2002 ISSN: 0027-8424**
- **SCHMIDT B ET AL: "SIMPLE ALGORITHM DERIVED FROM A GENO-/PHENOTYPIC DATABASE TO PREDICT HIV-1 PROTEASE INHIBITOR RESISTANCE" AIDS, LONDON, GB, vol. 14, no. 12, August 2000 (2000-08), pages 1731-1738, XP008015669 ISSN: 0269-9370**
- **SEVIN ANNE D ET AL: "Methods for investigation of the relationship between drug-susceptibility phenotype and human immunodeficiency virus type 1 genotype with applications to AIDS Clinical Trials Group 333" JOURNAL OF INFECTIOUS DISEASES, vol. 182, no. 1, July 2000 (2000-07), pages 59-67, XP009019956 ISSN: 0022-1899**
- **MEISEL PETER ET AL: "Prediction of metabolic activity from genotype: The gene-dose effect of N-acetyltransferase" THERAPEUTIC DRUG MONITORING, vol. 23, no. 1, February 2001 (2001-02), pages 9-14, XP009019868 ISSN: 0163-4356**

EP 1 636 727 B1

**(Cont. next page)**

- MEISEL P ET AL: "Relationship between human genotype and phenotype of N-acetyltransferase (NAT2) as estimated by discriminant analysis and multiple linear regression: 1. Genotype and N-acetylation in vivo" PHARMACOGENETICS, vol. 7, no. 3, 1997, pages 241-246, XP009019869 ISSN: 0960-314X
- SCHMIDT BARBARA ET AL: "Genotypic drug resistance interpretation systems: The cutting edge of antiretroviral therapy." AIDS REVIEWS, vol. 4, no. 3, 2002, pages 148-156, XP009019878 ISSN: 1139-6121 (ISSN print)
- BEERENWINKEL NIKO ET AL: "Geno2pheno: Estimating phenotypic drug resistance from HIV-1 genotypes." NUCLEIC ACIDS RESEARCH. 1 JUL 2003, vol. 31, no. 13, 1 July 2003 (2003-07-01), pages 3850-3855, XP002330990 ISSN: 1362-4962

**Description**

The present invention concerns methods and systems for analysis of drug resistance in HIV-1. More specifically, the invention provides methods for predicting drug resistance by correlating genotypic information with phenotypic profiles. The methods allow the identification of primary and secondary resistance-associated mutations for new and existing drugs and for calculating the contribution of mutations and combinations of mutations to resistance and hyper-susceptibility. The invention allows the design, optimization and assessment of the efficiency of a therapeutic regimen based upon the genotype of the disease affecting a patient.

**BACKGROUND**

Techniques to determine the resistance of HIV-1 to a therapeutic agent are becoming increasingly important. Many patients experience treatment failure or reduced efficacy over time. This is generally due to the virus mutating and/or developing a resistance to the treatment. As used herein, "HIV" is the human immunodeficiency virus, which is a retrovirus.

The various different anti-HIV-1 agents that have been developed over the years were initially administered to patients alone, as monotherapy. Though a temporary antiviral effect was observed, all the compounds lost their effectiveness over time. Research has now demonstrated that one of the main reasons behind treatment failure for all the antiviral drugs is the development of resistance of the virus to the drug (see, for example, Larder et al., 1989, Science, 246, 1155-8). This is largely due to the ability of HIV continuously to generate a number of genetic variants in a replicating viral population. These genetic changes generally alter the configuration of the HIV reverse transcriptase (RT) and protease (PR) molecules in such a way that they are no longer susceptible to inhibition by compounds developed to target them. If antiretroviral therapy is ongoing and if viral replication is not completely suppressed, the selection of genetic variants is inevitable and the viral population becomes resistant to the drug.

Since then, dual combination therapy, using drugs that target both HIV reverse transcriptase (RT) and protease (PR) molecules, has provided increased control of viral replication, and thus provided extended clinical benefit to patients. In recent years, however, it has become clear that even patients being treated with triple therapy including a protease inhibitor often eventually experience treatment failure.

Since patients in the developed world are generally prescribed cocktails of therapeutic drugs, not all HIV-1 infections originate with a wild type but with drug sensitive strains, from which drug resistance inevitably emerges. As such, with the increase in prevalence of drug resistant strains, there comes an increase in infections that actually begin with drug resistant strains. Infections with pre-existing drug resistance immediately reduce the drug options for drug treatment and emphasize the importance of drug resistance information to optimize initial therapy for these patients.

Moreover, as the number of available antiretroviral agents has increased, so has the number of possible drug combinations and combination therapies. It is therefore very difficult, if not impossible, for the physician to establish the optimal combination for an individual. Although there are many drugs available for use in combination therapy, the choices can quickly be exhausted and the patient can rapidly experience clinical progression or deterioration if the wrong treatment decisions are made. The key to tailored, individualized therapy lies in the effective profiling of the individual patient's virus population in terms of sensitivity or resistance to the available drugs. This requires the advent of truly individualized therapy.

There are certain solutions to this problem currently in use.

Phenotyping directly measures the actual sensitivity of a patient's pathogen or malignant cell to particular therapeutic agents. However, this can be slow, labor-intensive and thus expensive.

A second approach to measuring resistance involves genotyping tests that detect specific genetic changes (mutations) in the viral genome which lead to amino acid changes in at least one of the viral proteins, known or suspected to be associated with resistance. Although genotyping tests can be performed more rapidly, a problem with genotyping is that there are now over 100 individual mutations with evidence of an effect on susceptibility to HIV-1 drugs and new ones are constantly being discovered, in parallel with the development of new drugs and treatment strategies. The relationship between these point mutations, deletions and insertions and the actual susceptibility of the virus to drug therapy is extremely complex and interactive. An example of this complexity is the M184V mutation that confers resistance to 3TC but reverses AZT resistance. The 333D/E mutation, however, reverses this effect and can lead to dual AZT/3TC resistance.

Sophisticated interpretation is therefore required to predict what the net effect of these mutations might be on the susceptibility of the virus population to the various therapeutic agents. Custom algorithms such as rules-based computer algorithms have provided some assistance, for example, see International patent application WO01/79540. An overview of this type of technique is presented in Figure 1.

Beerenwinkel et al., PNAS (Jun 2002), 99(12), pp 8271-8276; Schmidt et al., AIDS (Aug 2000), 14(12), pp 1731-1738; and Sevin et al., Journal of Infectious Diseases, (Jul 2000), 182(1), pp 59-67; disclose methods for quantitating the individual contribution of a mutation or combination of mutations to the drug resistance phenotype exhibited by HIV based on different algorithms such as, respectively, decision trees, a rule-based approach and statistical analyses such as cluster analysis, recursive partitioning, linear discriminant analysis. In Schmidt et al., AIDS Reviews, 4(3), pp 148-156, further methods are reviewed.

Meisel et al., Therapeutic Drug Monitoring (Feb 2001), 23(1), pp 9-14; and Meisel et al., Pharmacogenetics (1997), 7(3), pp 241-246; disclose a method for predicting the metabolic activity phenotype from the mutation pattern of the NAT-2 gene by multiple linear regression analysis. The linear regression model describes a quantity $R_s$, the metabolic ratio built up by an error term (first term) and a sum of products built up from a mutation factor multiplied by a mutation-dependent resistance coefficient. However, given the nature of the NAT-2 genotypic patterns, the above methods do not consider the relationship between point mutations within a genotypic pattern. In particular, the quantitative prediction methods proposed are merely an addition of independent variables where effects such as antagonism or synergy between point mutations, insertions or deletions are not taken into account.

There remains a continuing need for the quantitative prediction of HIV drug susceptibility from viral genotype. In particular, there is a need for quantitative prediction methodologies like linear regression modelling which can grasp the complexity of the HIV-1 genotypic-to-phenotypic dynamics, i.e. combinatorial effects such as antagonism and synergism. Furthermore, because the majority of HIV patients have now been exposed to drug cocktails, it is thought that the disease-causing retroviruses tend to spontaneously generate mutations that have often co-evolved. This makes the analysis of which mutations are responsible for resistance to which drugs almost impossible using currently available techniques. It also means that mutations that contribute to resistance are being overlooked using the currently available analysis techniques.

It is therefore an aim of the present invention to provide methods for improving the interpretation of genotypic results.

It is a further aim of the invention to provide methods for determining (or predicting) a phenotype based on a genotype.

It is also a further aim of the invention to provide methods for predicting the resistance of an HIV variant of a particular genotype to a therapy or a therapeutic agent.

It is also an aim of the invention to predict resistance of a patient to therapy.

It is also an aim of the invention to provide methods to assess the effectiveness or efficiency of a therapy or to optimize a patient's therapy.

It is also an aim of the invention to identify novel HIV-1 mutations that are associated with resistance to particular drug therapies or combination therapies.

## SUMMARY OF THE INVENTION

A solution to these problems involves new methods for measuring drug resistance by correlating genotypic information with phenotypic drug resistance profiles measured experimentally.

According to a first aspect of the invention, there is provided a method for quantitating the individual contribution of a mutation or combination of mutations to the drug resistance phenotype exhibited by HIV, said method comprising the steps of

a) performing a linear regression analysis using data from a dataset of matching genotypes and phenotypes, whereby the log fold resistance, pFR, is modelled as the sum of all the individual resistance contributions for each of the mutations or combinations of mutations that occur in HIV according to the following equation;

$$pFR = \beta_A M_A + \beta_B M_B + \cdots + \beta_Z M_Z + \varepsilon$$

wherein each individual resistance contribution is calculated by multiplying a mutation factor, $M_A$, $M_B$, ..., $M_Z$, for each mutation or combination of mutations by a resistance coefficient $\beta_A$, $\beta_B$, ..., $\beta_Z$;

wherein the mutation factor assigned to each mutation or combination of mutations reflects the degree to which that mutation or combination of mutations is present in the HIV strain and, if present, to which degree the mutation is present in a mixture;

wherein each resistance coefficient reflects the contribution of the mutation or combination of mutations to the fold resistance exhibited by the strain;

and wherein the error term $\varepsilon$, represents the difference between the modelled prediction and the experimentally determined measurement.

This method involves a data driven technique for quantitative drug susceptibility prediction. This method uses a multiple linear regression model to estimate coefficient values that accurately reflect the contribution made by a particular HIV mutation or combination of mutations to resistance to a particular drug. Repeating the method for each candidate therapeutic drug allows the compilation of a global picture of drug resistance exhibited by a particular HIV strain.

This method has allowed the identification of mutations hitherto unrecognized as having an effect on drug resistance in HIV. The method also allows the identification of primary (single mutations) and secondary (the co-occurrence of two mutations) or higher order terms resistance-associated mutations for new and existing drugs, which embrace the antagonistic and synergistic phenomena. Accordingly, a further aspect of the invention provides a method of identifying a mutation that affects the degree of drug resistance exhibited by an HIV strain using a method according to the first aspect of the invention.

The method of the first aspect of the invention is also advantageous over current methods since it allows the quantitative, purely data-driven, objective assessment of the contribution of mutations and combinations of mutations to drug resistance. The method also allows the de-convolution of the individual contribution made by particular mutations to the drug resistance phenotype. Unlike existing methods, the method is able to correct for correlating mutations that on the face of it appear to affect drug resistance, but which in fact only correlate in their occurrence with resistance causing mutations and are themselves phenotypically silent.

The method has allowed the design of an automated computational technique for the prediction of the drug resistance profile possessed by a particular HIV strain infecting a patient. The methods thus allow the determination of a patient phenotype without having to perform any phenotypic testing whatsoever. This has clear ramifications for the bespoke design, optimisation and assessment of strategies for individual patient therapy based upon the genotype of the infecting agent.

**DESCRIPTION**

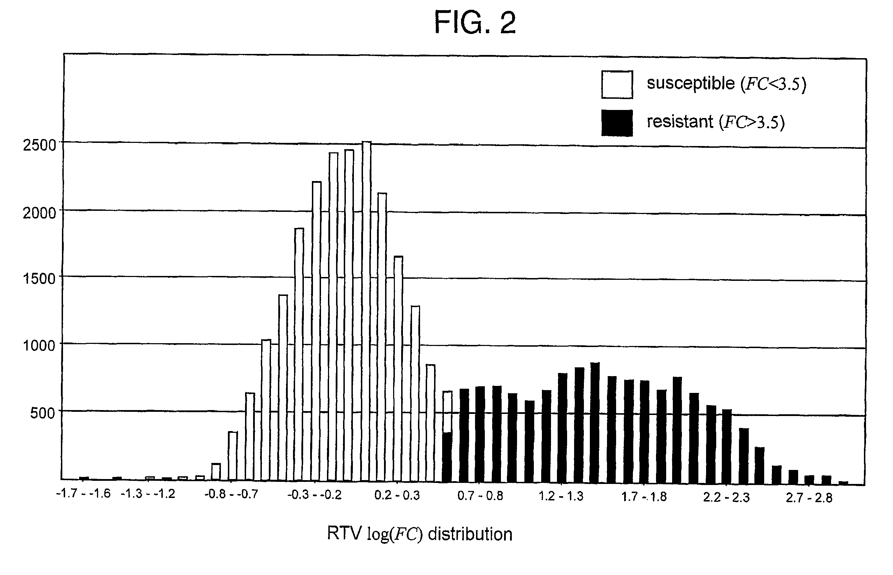

In any population of HIV variants, there is a wide distribution of drug resistance phenotypes for any particular drug, ranging from hyper-susceptibility to strong resistance (see Figure 2). The expression "drug resistance phenotype" means the resistance of an HIV virus to a tested therapy, therapeutic agent or drug. The term "resistance" as used herein, pertains to the capacity of resistance, sensitivity, susceptibility, hyper-susceptibility or effectiveness of a therapy against a disease. The term "therapy" includes but is not limited to a drug, pharmaceutical, or any other compound or combination of compounds that can be used in therapy or therapeutic treatment of HIV. This distribution of drug resistance reflects the large number of different genotypes that are present in the population. Some variants may only have one mutation that is correlated with drug resistance, whilst others will have several or numerous such mutations, each of which may impart its own contribution to the drug resistance phenotype.

Adding an additional level of complication are the phenomena of antagonism, synergy and enhancement, where certain mutations may add to or detract from the effect of other mutations in a manner not predictable from studying the effects of the individual mutations alone. Highly correlated mutations are also problematic. These are mutations that almost always co-occur in a strain, but only one of the mutations actually has an effect on drug resistance. For example, when one of these 2 mutations has an effect on resistance and the other mutation does not (this mutation might for example be highly correlated with the resistance mutation because it affects the replication rate of the virus), the effect can erroneously be assigned to either one of the mutations.

Examples of mutations known or suspected to influence the sensitivity of HIV to drug therapy may be found on the internet at http://hiv-web.lanl.gov; http://hivdb.stanford.edu/hiv/; or http://www.viral-resistance.com.

In HIV, two sections of the genome are generally studied: Protease (PR) and Reverse Transcriptase (RT). The methods of the present invention can equally be applied to other sections of the HIV genome such as Integrase (IN). A mutation is presented as a number referring to the position in the protein, followed by the amino acid(s) on that position, if it differs from the amino acid in the HXB2 HIV reference. In the terms included above, the mutations are represented as "A", "B", ..."Z".

Mixtures reflect the diversity of the HIV population in a sample. It means that on that position two subsets of the population have a different amino acid. Mixtures are denoted by separating amino acids with the '/' character: 65K/R (mixture of 'K' and 'R' at position 65).

When more than two amino acids are found on a certain position in subsets of the population, the dummy amino acid 'X' is used.

Insertions are denoted by adding the insert position behind a dot: 69.2S (an insert of 'S' at insert position 2). Deletions are denoted by a minus sign: 69-.

Examples of mutations present in the RT domain of HIV conferring resistance to a reverse transcriptase inhibitor include 69C, 69V, 69T, 75A, 1011, 103T, 103N, 184T, 188H, 190E, 219N, 219Q, 221Y, 2211, and 233V. Additional examples of mutations present in the protease (PR) domain of HIV conferring resistance to a reverse transcriptase inhibitor include 24M, 48A, and 53L. A mutation may affect resistance alone or in combination with other mutations.

For the purposes of the invention, the mutations identified should be associated with resistance or susceptibility to drug therapy, for example an antiretroviral drug. The degree to which a particular mutation pattern may affect resistance may be determined by one of skill in the art, for example, using the phenotypic resistance monitoring assay such as, the ANTIVIROGRAM® (Virco, Belgium) (see WO97/27480). In this methodology, resistance is determined with respect to a laboratory reference strain HIVLAI/IIIB. The difference in $IC_{50}$ (the concentration of drug required to reduce the virus' growth in cell culture by 50%) between the patient sample and the reference viral strain is determined as a quotient. This fold change in $IC_{50}$ is reported and indicative of the resistance profile of a certain drug. Based on the changes in $IC_{50}$, cut-off values have been established to distinguish a sample from being sensitive or resistant to a certain drug.

Various projects are underway to compile data relating to the correspondence of certain mutations with drug resistance phenotype, and these generally lead to the generation of relational databases of tables that illustrate the matching genotype / resistance phenotype for various antiretroviral drugs. Such databases bring together the knowledge of both a genotypic and phenotypic database. The phenotypic database contains phenotypic resistance values for HIV to at least one therapy, preferably multiple drug therapies. For example, the phenotypic resistance values of tested HIV viruses, with a fold resistance determination compared to the reference HIV virus (wild type).

The dataset used herein is a dataset developed by the Applicant, which consists of a set of matching genotype / phenotype measurements with possible multiple phenotype measurements per genotype. However, any similar dataset may be used, provided that there are sufficient entries for each genotype / phenotype measurement for the data to be significant. In the Virco dataset, the mutations are defined relative to HXB2 at amino acid level.

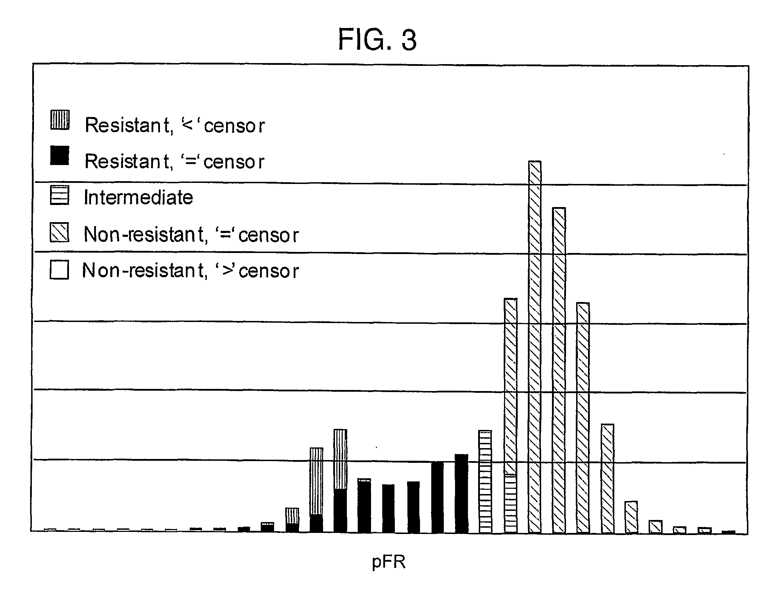

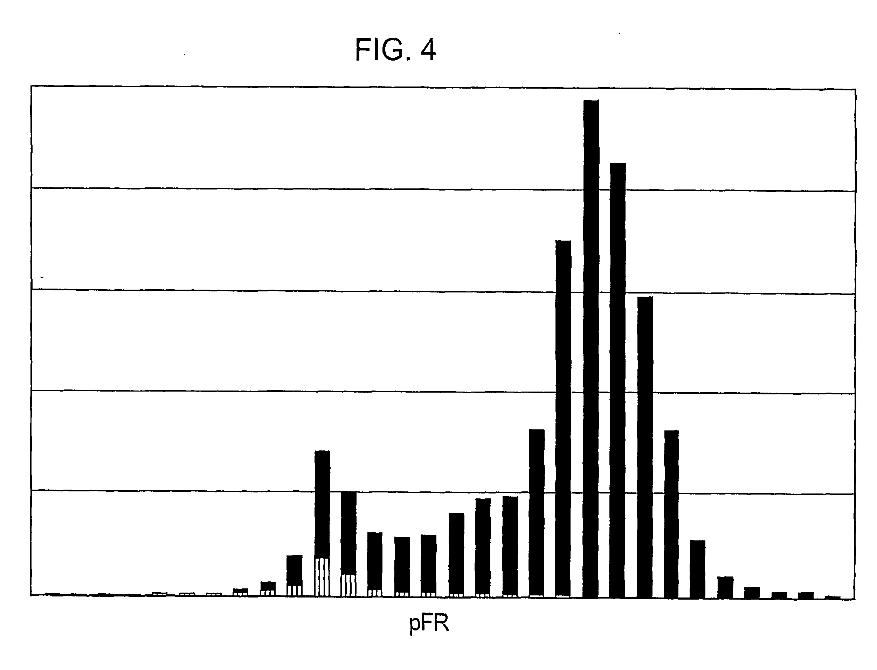

The phenotypes are presented as *pFR* values, where *pFR* is equal to - *log (FR)*, where *FR* denotes the Fold Resistance. Negative *pFR* values thus denote resistance and positive values denote hyper-susceptibility. For example, a *pFR* value of -1.0 is equal to 10-fold resistance. An example of the pFR distribution for Saquinavir (SQV) is shown in Figure 3. Figure 4 shows the pFR distribution for the "48V" mutation on SQV. It is clear from this that the 48V subset does not behave the same as the whole dataset.

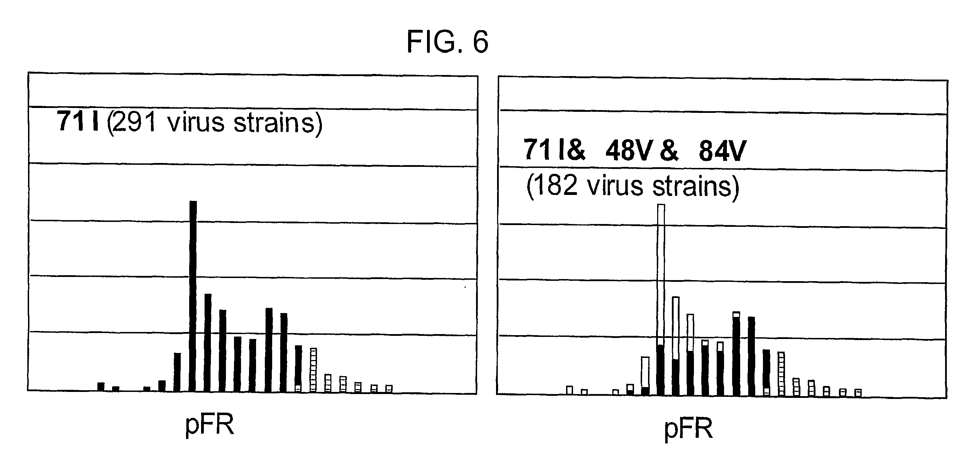

The problems of unwanted correlations between mutations where not all correlated mutations contribute to the drug resistance phenotype are illustrated in Figure 6. Here, the left hand panel shows the pFR distribution for the 71I mutation. When the effects of mutations 48V and 84V are removed (right hand panel), the pFR in the distribution of variants is markedly increased (less drug resistance).

According to the invention, the predicted fold resistance of an HIV strain of a particular genotype may be calculated by summing the individual resistance contributions for each of the mutations or combinations of mutations in the mutation pattern of that genotype. The method uses linear regression models, so that the phenotype prediction, *pFR* is calculated in the following equation (1):

$$pFR = \beta_A M_A + \beta_B M_B + \cdots + \beta_Z M_Z + \varepsilon$$

The independent variables $M_A$, $M_B$, ..., $M_Z$, are referred to herein as mutation factors, each of which reflects the degree to which the mutation or combination of mutations is present in the HIV strain and, if present, whether or not the mutation is present in a mixture.

The resistance coefficients $\beta_A$, $\beta_B$, ..., $\beta_Z$ represent the contribution to the total pFR prediction for each single mutation.

Each mutation factor $M_i$ thus represents the presence or absence of the corresponding mutation and each coefficient $\beta_i$ represents the contribution to the pFR change for that specific mutation.

The mutation factor may take into account 1st order terms (single mutations) as well as 2nd order terms (the co-occurrence of two mutations) and in general nth order terms. For example, 2nd order terms take the form:

$$\beta_{AB} M_{AB}$$

The independent variable MAB represents the co-occurrence of mutations A and B and the coefficient $\beta_{AB}$ represents the synergy or antagonism between mutations A and B. When the mutation factor embraces nth order terms, thus the co-occurrence of two ore more mutations, the terms take the form:

$$\beta_n M_n$$

wherein $M_n$ represents the co-occurrence of one mutation with other one or more mutations -such as duplets, triplets, quadruples, etc. and the coefficient $\beta_n$ represents the synergy or antagonism between the one mutation with the other one or more mutations; thus *n* will apply for instance to the combinations of mutations, AB, ABC, ABCD, BC, ACD, etc.

As such, the linear regression model may take the following equation:

$$pFR = \beta_A M_A' + \beta_B M_B + \beta_n M_n + \cdots + \beta_Z M_Z + \varepsilon$$

Higher order terms affect for interactions between mutations:

- antagonism or reversal: positive *pFR* shifts for mutation couples.
- synergy or enhancement: extra negative *pFR* shift for mutation couple.

For example, consider the following (artificial) model:

| Mutation | Coefficient |
|----------|-------------|
| 84V | -0.46 |
| 50V | -0.92 |
| 54M | -0.64 |
| 88S | 0.63 |
| 90M | -0.16 |
| 46L | -0.19 |
| 46L & 84V | -0.09 |

Consider a virus with following mutations: 3I, 46L, 84V and 90M. Applying equation (1), this virus will have a pFR prediction:

$$pFR = \beta_{46L} \cdot 1 + \beta_{84V} \cdot 1 + \beta_{90M} \cdot 1 + \beta_{46L,84V} \cdot 1 = -0.9$$

or almost 8-fold resistance, i.e. *pFR* = -logFR.

Note that in the model F and A are synergistic since their co-occurrence decreases the *pFR* by an extra -0.09.

The error term is ε, which is the difference between the prediction and the measurement. This error term contains both the *measurement error* on the phenotype measurement and a *model error* (if the underlying model has higher order terms that are not taken into account in the regression model).

Mutation factors for single mutations ($M_A$, $M_B$, ..., $M_Z$) are calculated as follows:

if the mutation is present in the HIV strain, a positive mutation factor is assigned;

if the single mutation is not present, the mutation factor assigned is zero;

if the single mutation is present in a mixture, an averaged positive mutation factor is assigned.

Conveniently, mutation factors range between 0 and 1 where 0 means not present and 1 means present. Values between 0 and 1 means that the mutation is present in a mixture. Accordingly, a positive mutation factor is assigned the value 1.

Mixtures are modelled as causing the average shift of its constituent mutations. Since methods for the quantitation of the precise proportions of mixtures to wild type are expensive and time-consuming, mixture with wild type may conveniently be treated as causing half the pFR shift of the resistance mutation (mutation factor = 0.5). However, as the skilled reader will appreciate, a more precise mutation factor may be assigned if the true proportion in the mixture is known.

Mutation factors for double mutations *(M$_{AB}$ etc.)* are calculated as follows;

if both the mutations are present in the HIV strain, a positive mutation factor is assigned (conveniently, the value 1);

if neither of the mutations are present, the mutation factor assigned is zero;

if both mutations are present and one mutation is present in a mixture, an averaged positive mutation factor is assigned (conveniently, 0.5);

if both mutations are present in a mixture, a reduced averaged positive mutation factor is assigned (in this example, 0.25). The factor 0.25 is the product of the M-factors of both the single constituent mutations. This is the result of the assumption that these mixtures are independent of each other. Of course, this is an approximation, since in a real blood sample, the mixtures are not independent of each other. For example, if only 2 viruses were present, virus A (no mutations) for 70% and virus B (mutations 46I and 84V) for 30%, then a mixture would be detected on both positions 46 and 84. If these concentrations were known, it would be possible to fine tune the mutation factor of 0.25. If this information is not available, the best statistical guess is 0.5*0.5: this being the average value that would be measured for the mutation couple being present for a population of samples that have these mixtures on 46 and 84 in all possible concentrations.

Similarly, the mutation factors for triple mutations $(M_{ABC}$ etc.) shall be calculated as follows:

if the three mutations are present in the HIV strain, a positive mutation factor is assigned (conveniently, the value 1);

if neither of the three mutations are present, the mutation factor assigned is zero;

if the three mutations are present and one mutation is present in a mixture, an averaged positive mutation factor is assigned (conveniently, 0.5);

if the three mutations are present and two mutations are present in a mixture, a reduced averaged positive mutation factor is assigned (in this example, 0.25). The factor 0.25 is the product of the M-factors of the two single constituent mutations present in the mixture;

if the three mutations are present in a mixture, a reduced averaged positive mutation factor is assigned (in this example, 0.125). The factor 0.125 is the product of the M-factors of the three single constituent mutations.

The calculation of the mutation factors for higher order terms shall take the same principle.

Calculation of the resistance coefficient $(\beta_A, \beta_B, ..., \beta_Z, \beta_{AB})$ is performed by evaluating the dataset for the drug phenotype reported for each mutation or combination of mutations.

The problem of unwanted correlations has been discussed above. Unwanted correlations are removed according to the methods of the invention.

One way to do this is to use an algorithm that has been developed by the inventors to track the change in pFR as the effects of individual mutations or combinations of mutations are removed from the dataset. The effect of each mutation or combination of mutations is thus separated out. The methodology follows mutation trajectories towards the global average as the effects of individual mutations or combinations of mutations are removed. The steps are as follows:

a) calculate average pFR for all mutations with a sufficient count in the database to be significant;

b) determine the extremes (maximum, minimum), and select the mutation with the pFR furthest away from the global average;

c) remove all virus strains that have the selected mutation and reiterate from step a);

d) stop when the selected mutation in step b) has an average pFR that approximates to the global average.

In this manner, mutations that do not cause resistance, but which are often present with mutations that do cause resistance will have a higher average pFR (less resistance). Removing the virus strains with a certain resistance causing mutation results in an increase of the average pFR for correlating mutations.

A suitable threshold at which a count in the database becomes sufficiently significant will be apparent to the skilled reader and will be dependent on the database size. For example, thresholds of 5, 10, 15, 20, 25, 30 or more may be suitable. In the examples discussed herein, a threshold of 20 times was used.

By an "average pFR that approximates to the global average" is meant that the average pFR is within a fraction of the standard deviation of the remaining population. A convenient fraction ranges between about 0.3 and 0.5.

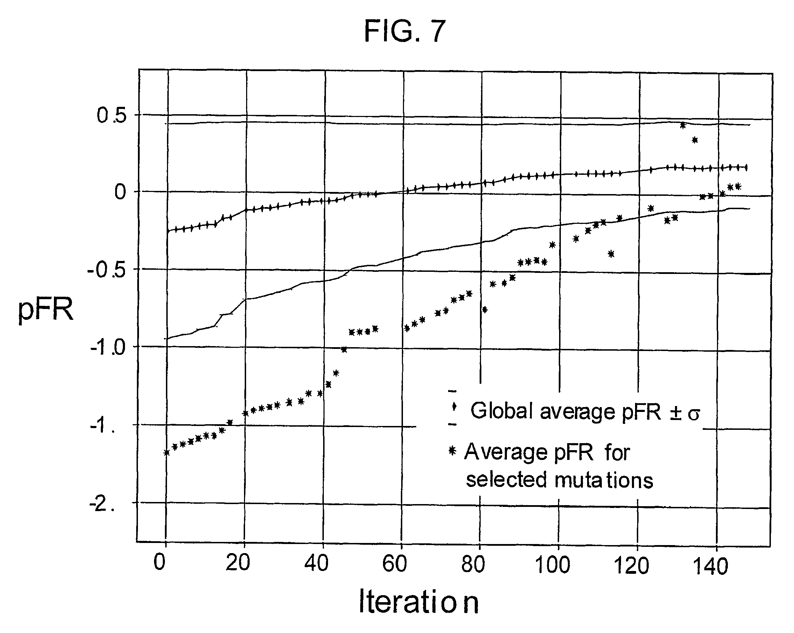

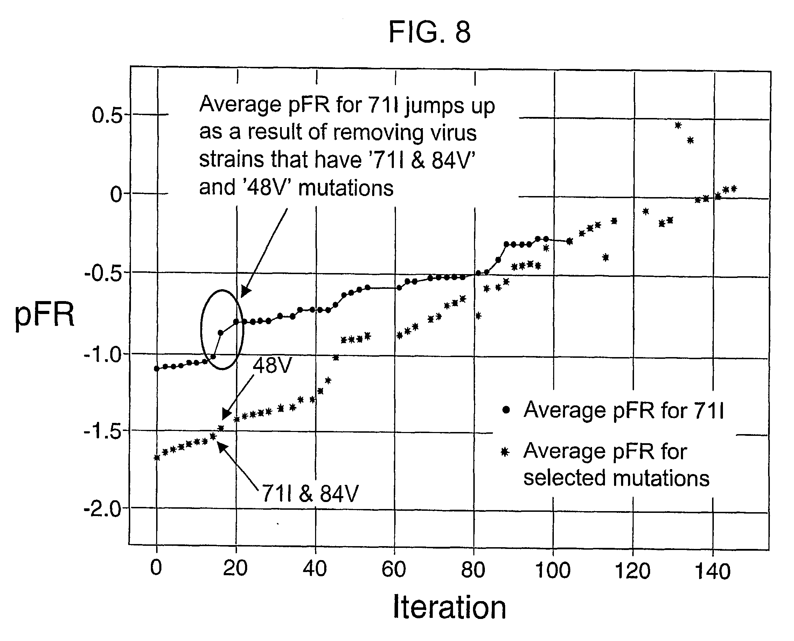

A comparison of the change in the global average pFR with the change in the average pFR for selected mutations with increasing iterations of the algorithm is shown in Figure 7. Figure 8 shows an example, where the average pFR for 71I (unwanted correlation) jumps up as a result of removing from the dataset virus strains that have "71I & 84V" and "48V" mutations.

An alternative, analogous methodology for removing unwanted correlations is as follows; this is an extension of the mutation trajectories algorithm discussed above. The steps of this method are as follows:

a) calculate correlation coefficient between all mutations (with a sufficient count in the database) and the pFR;

b) determine the extremes (maximum, minimum), and select the mutation with the highest (absolute value of) correlation coefficient;

c) calculate a linear model for the pFR with the selected mutation(s) (from step b), all previous iterations);

d) take the residue (pFR minus the predicted value from the model);

e) calculate correlation coefficient between all mutations (with a sufficient count in the database) and the residue;

f) determine the extremes (maximum, minimum), and select the mutation with the highest (absolute value of) correlation coefficient;

g) calculate a linear model for the pFR with the selected mutation(s) (from step f), all previous iterations); and

h) reiterate from step d);

i) stop when the selected mutation in step g) has a correlation coefficient that approximates to zero.

As with the mutation trajectories algorithm described above, the effect of mutations that do not themselves cause resistance, but which are often present with mutations that do cause resistance, is excluded and thus does not distort the real values.

In a more preferred methodology for removing unwanted correlations, a stepwise selection regression may be applied, which method selects the variable with the highest effect. The steps of this method are as follows:

a) perform a first order regression from the list of mutations that occur in the dataset;

b) calculate the p-value for all mutations;

c) select the mutation with the lowest p-value and add it to the model;

d) re-calculate the regression model;

e) reiterate from step b);

f) stop when the re-calculation of the p-values of step b) gives no significant values anymore.

Usually, this methodology is run in statistical software packages, which iteratively model the residue from the previous regression as the dependent variable.

$$pFR = Intercept_1 + \beta_A M_A + \varepsilon_1$$

$$\varepsilon_1 = Intercept_2 + \beta_B M_B + \varepsilon_2$$

$$\varepsilon_2 = Intercept_3 + \beta_C M_C + \varepsilon_3$$

$$\cdots$$

The p-value for a given mutation is the probability of rejecting the true null hypothesis, where the null hypothesis is defined as follows: the coefficient of that parameter equals zero. In other words, the p-value is the probability that the real coefficient for a certain parameter is zero, while the model predicts a coefficient different from zero.

The expression "significant value" for the p-values refers to the mutations with a p-value that is lower than the threshold selected by the user. This threshold may be determined as follows: the user shall create linear models for a whole range of combinations of p-values (a p-value for the first-order iteration and a p-value for the second-order iteration). For each combination, the mean squared error (MSE) of the corresponding model is calculated on unseen data. The combination of p-values that results in the model with the lowest MSE, i.e. the combination for which the model gives the best predictions, is chosen.

In further preferred embodiments of the invention, problems of small datasets for particular mutations or combinations of mutations are dealt with by applying the method recursively to the set of virus strains that exhibit those particular mutations or combinations of mutations.

In still further preferred embodiments of the invention, the following additional correlations are taken into account:

● multiple entries of the same virus strain (or virus strains grown from the same stock solution) that cause unwanted correlations;

● censored values in genotype / phenotype database (for example, $EC_{50}$ value = '< 1μM'). These are phenotypes beyond the assay range, thus when the phenotypic value is smaller than the measurable range, a '<'-censor is applied to that value. Analogously, a '>'-censor is applied to the value, if it is higher than the measurable range.

Preferably, censored values are dealt with by attempting to construct a model that is consistent from extrapolations. Censored values are thus modeled by replacing the censored value by a maximum likelihood estimation, assuming knowledge of the standard deviation of the measurement error.

A preferred technique for the generation of a maximum likelihood estimation is as follows:

a) calculate a linear regression model without censored values;

b) use the phenotypic measured value $V_0$ as if the censor was " = ", e.g. when a result is expressed as -log FR < 4, we will treat $V_0$ as -log FR =4;

c) look at the prediction P from the model and apply either:

Case '<'-censor:

■ $P < V_0 - 0.798\,\sigma$ (center of gravity of half Gaussian distribution)

o Remove value from training data for next iteration

■ $V_0 - 0.798\,\sigma \leq P < V_0$

o Use $V' = V_0 - 0.798\,\sigma$ for next iteration

■ $V_0 \leq P$

o Use V' centre of gravity of tail (<V) of a normal distribution N (P, σ) as value for next iteration.

Case '>'-censor:

■ $P > V_0 + 0.798\,\sigma$ (center of gravity of half Gaussian distribution)

o Remove value from training data for next iteration

■ $V_0 + 0.798\,\sigma \geq P > V_0$

o Use $V' = V_0 - 0.798\,\sigma$ for next iteration

■ $V_0 \geq P$

o Use V' centre of gravity of tail (>V) of a normal distribution N (P, σ) as value for next iteration.

d) calculate a linear regression model and for the censored values in the linear regression model, either remove the data-point from the training set, or use V' instead of the censored phenotypes measurement, as described in step c);

e) re-iterate from step b) until the prediction converges.

Accordingly, for each iteration, when the prediction and measurement contradict, censored values are taken into account. When the prediction and measurement are strongly consistent, censored values are disregarded, on the basis that no further information is provided and their inclusion has no additional value.

In one preferred embodiment of this aspect of the invention, the number of calculations necessary in the linear regression analysis may be reduced. The computational power and memory requirement that is currently generally available is insufficient to allow a full second order model to be evaluated for a large dataset, based on all possible single mutations and second order terms, since the number of terms increases quadratically with the number of mutations considered. This number increases with a larger dataset since more rare mutations are in a large database.

In order to reduce the amount of terms, a first order regression may be performed from the list of mutations that occur in the dataset above a threshold number of times. A suitable threshold at which a count in the database becomes sufficiently significant will be apparent to the skilled reader and will be dependent on the database size. For example, thresholds of 5, 10, 15, 20, 25, 30 or more may be suitable. In the examples discussed herein, a threshold of 20 times was used. The significant terms from this first order regression are withheld and the list of these terms is then used to perform a second order regression. In the second order regression only the single mutations and combinations of mutations are used that were found significant in the first order model. Again, a threshold significance will be apparent to the skilled reader - an example is if the probability that the real value of the term is 0, is smaller than 0.001.

For example, a first order regression performed on the matching genotype / phenotype dataset for indinavir (34,445 measurements) for those mutations that occur at least 20 times results in a first order model that withholds a list of 94 single mutations that are considered significant.

This list is then used as a starting list for a second order regression. It should be noted that it may be advantageous to exclude certain very common mutations from the calculation. 3I is one example. The reason is that a mutation must occur at least a threshold number of times and the inverse also has to be true: the count of viruses *not* having the mutation 3I or the couple *not* 3I and another mutation should also be above the threshold value (e.g. 20). Taking this into account results in excluding 3I from the regression in practice.

In the second order regression, all the single mutations and all couples of mutations from the list are used as potential terms. The significant terms are then withheld by the regression algorithm.

According to a further aspect of the invention, there is provided a method of calculating the quantitative contribution of a mutation pattern to the drug resistance phenotype exhibited by an HIV strain, said method comprising the steps of:

a) obtaining a genetic sequence of said HIV strain;

b) identifying the pattern of mutations in said genetic sequence, wherein said mutations are associated with resistance or susceptibility to drug therapy; and

c) calculating the fold resistance of the HIV strain as compared to the wild type HIV strain by performing a linear regression analysis, whereby the log fold resistance, pFR, is modelled as the sum of all the individual resistance contributions for each of the mutations or combinations of mutations that occur in said HIV strain according to the following equation;

$$pFR = \beta_A M_A + \beta_B M_B + \cdots + \beta_Z M_Z + \varepsilon$$

wherein each individual resistance contribution is calculated by multiplying a mutation factor, $M_A$, $M_B$, ..., $M_Z$, for each mutation or combination of mutations by a resistance coefficient $\beta_A$, $\beta_B$, ..., $\beta_Z$;

wherein the mutation factor assigned reflects the degree to which the mutation or combination of mutations is present in the HIV strain and, if present, to which degree the mutation is present in a mixture; wherein each resistance coefficient reflects the contribution of the mutation or combination of mutations to the fold resistance exhibited by the strain;

and wherein the error term $\varepsilon$, represents the difference between the modelled prediction and the experimentally determined measurement.

As the skilled reader will appreciate, the fold resistance of the HIV strain may be calculated using any one of the embodiments of the invention referred to above.

In the first step of this method, the genetic sequence of an HIV strain should be obtained. Normally, this will be the genetic sequence of an HIV strain with which a patient is infected, although the sequence may be a theoretical sequence, for example for purposes of *in silico* modelling.

The method may thus be used as a diagnostic method for predicting the fold resistance exhibited by a particular HIV strain with which a patient is infected. According to other preferred embodiments, the method may be used for assessing the efficiency of a patient's therapy or for evaluating or optimising a therapy. The method may be performed for each drug or combination of drugs currently being administered to the patient so as to obtain a series of drug resistance phenotypes and thus to assess the effect of a plurality of drugs or drug combinations on the predicted fold resistance exhibited by the HIV strain with which the patient is infected.

A "patient" may be any organism, particularly a human or other mammal, suffering from HIV or AIDS or in need or desire of treatment for such disease. A patient includes any mammal and particularly humans of any age or state of development.

To obtain an HIV strain from a patient, a biological sample will need to be obtained from the patient. A "biological sample" may be any material obtained in a direct or indirect way from a patient containing HIV virus. A biological sample may be obtained from, for example, saliva, semen, breast milk, blood, plasma, faeces, urine, tissue samples, mucous samples, cells in cell culture, cells which may be further cultured, etc. Biological samples also include biopsy samples.

The genetic sequence of an HIV strain may be evaluated by a number of suitable means, as will be clear to those of skill in the art. Most suitable will be techniques that allow for specific nucleic acid amplification, such as the polymerase chain reaction (PCR), although other techniques such as restriction fragment length polymorphism (RFLP) analysis will be equally applicable.

Nucleic acid sequencing then allows the analysis of the mutation pattern in a particular nucleic acid sequence, either by classical nucleic sequencing protocols e. g. extension chain termination protocols (Sanger technique; see Sanger F., Nicher., Coulson A. Proc. Nat. Acad. Sci. 1977,74, 5463-5467) or chain cleavage protocols. Such methods may employ such enzymes as the Klenow fragment of DNA polymerase I, Sequenase (US Biochemical Corp, Cleveland, OH), Taq polymerase (Perkin Elmer), thermostable T7 polymerase (Amersham, Chicago, IL), or combinations of polymerases and proof-reading exonucleases such as those found in the ELONGASE Amplification System marketed by Gibco/BRL (Gaithersburg, MD). Preferably, the sequencing process may be automated using machines such as the Hamilton Micro Lab 2200 (Hamilton, Reno, NV), the Peltier Thermal Cycler (PTC200; MJ Research, Watertown, MA) and the ABI Catalyst and 373 and 377 DNA Sequencers (Perkin Elmer). Particular sequencing methodologies have been developed further by companies such as Visible Genetics. Any of the novel approaches developed for unraveling the sequence of a target nucleic acid, either now or in the future will be perfectly applicable to the analysis of sequence in the present invention (including but not limited to mass spectrometry, MALDI-TOF (matrix assisted laser desorption ionization time of flight spectroscopy) (see Graber J, Smith C., Cantor C. Genet. Anal. 1999, 14, 215-219) chip analysis (hybridization based techniques) (Fodor S P ; Rava R P ; Huang XC; Pease A C ; Holmes C P ; Adams C L Nature 1993, 364, 555-6) It should be appreciated that nucleic acid sequencing covers both DNA and RNA sequencing.

Once the genetic sequence of the HIV strain is known, the pattern of mutation must be identified in the sequence. The term "mutation" as this is used herein, encompasses both genetic and epigenetic mutations of the genetic sequence of wild type HIV. A genetic mutation includes, but is not limited to, (i) base substitutions: single nucleotide polymorphisms, transitions, transversions, substitutions and (ii) frame shift mutations: insertions, repeats and deletions. Epigenetic mutations include, but are not limited to, alterations of nucleic acids, e. g., methylation of nucleic acids. One example includes (changes in) methylation of cytosine residues in the whole or only part of the genetic sequence. In the present invention, mutations will generally be considered at the level of the amino acid sequence, and comprise, but are not limited to, substitutions, deletions or insertions of amino acids.

The "control sequence" or "wild type" is the reference sequence from which the existence of mutations is based. A control sequence for HIV is HXB2. This viral genome comprises 9718 bp and has an accession number in Genbank at NCBI M38432 or K03455 (gi number : 327742).

Identifying a mutation pattern in a genetic sequence under test thus relates to the identification of mutations in the genetic sequence as compared to a wild type sequence, which lead to a change in nucleic acids or amino acids or which lead to altered expression of the genetic sequence or altered expression of the protein encoded by the genetic sequence or altered expression of the protein under control of said genetic sequence.

A "mutation pattern" comprises at least one mutation influencing sensitivity of HIV to a therapy. As such, a mutation pattern may consist of only one single mutation. Alternatively, a mutation pattern may consist of at least two, at least three, at least four, at least five, at least six, at least seven, at least eight, at least nine or at least ten or more mutations. A mutation pattern is thus a list or combination of mutations or a list of combinations of mutations. A mutation pattern of any particular genetic sequence may be constructed, for example, by comparing the tested genetic sequence against a wild type or control sequence. The existence of a mutation or the existence of one of a group of mutations can then be noted.

One way in which this may be done is by aligning the genetic sequence under test to a wild type sequence noting any differences in the alignment. Typical alignment methods include Smith-Waterman (Smith and Waterman, (1981) J Mol Biol, 147: 195-197), Blast (Altschul et al. (1990) J Mol Biol., 215(3): 403-10), FASTA (Pearson & Lipman, (1988) Proc Natl Acad Sci USA; 85(8): 2444-8) and, more recently, PSI-BLAST (Altschul et al. (1997) Nucleic Acids Res., 25(17): 3389-402). It may in some circumstances be preferable to generate alignments using a multiple alignment program, such as ClustalW (Thompson et al., 1994, NAR, 22(22), 4673-4680). Other suitable methods will be clear to those of skill in the art (see also "Bioinformatics: A practical guide to the analysis of genes and proteins" Eds. Baxevanis and Ouellette, 1998, John Wiley and Sons, New York). A practical example of multiple sequence alignment is the construction of a phylogenetic tree. A phylogenetic tree visualizes the relationship between different sequences and can be used to predict future events and retrospectively to devise a common origin. This type of analysis can be used to predict a similar drug sensitivity for a sample but also can be used to unravel the origin of different patient sample (i. c. the origin of the viral strain).

In this manner, therefore, the pattern of mutations in the genetic sequence can be identified, wherein said mutations are associated with resistance or susceptibility to drug therapy exhibited by the HIV strain tested. The mutation pattern may influence sensitivity to a specific therapy, e. g., a drug, or a group of therapies. The mutation pattern may, for example, increase and/or decrease resistance of the HIV strain to a therapy. Particular mutations in the mutation pattern, may also, for example, enhance and/or decrease the influence of other mutations present in the genetic sequence that effect sensitivity of the HIV strain to a therapy.

A still further aspect of the invention relates to a computer apparatus or computer-based system adapted to perform any one of the methods of the invention described above, for example, to quantify the individual contribution of a mutation or combination of mutations to the drug resistance phenotype exhibited by HIV, or to calculate the quantitative contribution of a mutation pattern to the drug resistance phenotype exhibited by an HIV strain.

In a preferred embodiment of the invention, said computer apparatus may comprise a processor means incorporating a memory means adapted for storing data; means for inputting data relating to the mutation pattern exhibited by a particular HIV strain; and computer software means stored in said computer memory that is adapted to perform a method according to any one of the embodiments of the invention described above and output a predicted quantified drug resistance phenotype exhibited by an HIV strain possessing said mutation pattern.

A computer system of this aspect of the invention may comprise a central processing unit; an input device for inputting requests; an output device; a memory; and at least one bus connecting the central processing unit, the memory, the input device and the output device. The memory should store a module that is configured so that upon receiving a request to quantify the individual contribution of a mutation or combination of mutations to the drug resistance phenotype exhibited by HIV, or to calculate the quantitative contribution of a mutation pattern to the drug resistance phenotype exhibited by an HIV strain, it performs the steps listed in any one of the methods of the invention described above.

In the apparatus and systems of these embodiments of the invention, data may be input by downloading the sequence data from a local site such as a memory or disk drive, or alternatively from a remote site accessed over a network such as the internet. The sequences may be input by keyboard, if required.

The generated results may be output in any convenient format, for example, to a printer, a word processing program, a graphics viewing program or to a screen display device. Other convenient formats will be apparent to the skilled reader.

The means adapted to quantify the individual contribution of a mutation or combination of mutations to the drug resistance phenotype exhibited by HIV, or to calculate the quantitative contribution of a mutation pattern to the drug resistance phenotype exhibited by an HIV strain will preferably comprise computer software means. As the skilled reader will appreciate, once the novel and inventive teaching of the invention is appreciated, any number of different computer software means may be designed to implement this teaching.

According to a still further aspect of the invention, there is provided a computer program product for use in conjunction with a computer, said computer program comprising a computer readable storage medium and a computer program mechanism embedded therein, the computer program mechanism comprising a module that is configured so that upon receiving a request to quantify the individual contribution of a mutation or combination of mutations to the drug resistance phenotype exhibited by HIV, or to calculate the quantitative contribution of a mutation pattern to the drug resistance phenotype exhibited by an HIV strain, it performs the steps listed in any one of the methods of the invention described above.

The invention further relates to systems, computer program products, business methods, server side and client side systems and methods for generating, providing, and transmitting the results of the above methods.

The invention will now be described by way of example with particular reference to a specific algorithm that implements the process of the invention. As the skilled reader will appreciate, variations from this specific illustrated embodiment are of course possible without departing from the scope of the invention.

**BRIEF DESCRIPTION OF THE FIGURES**

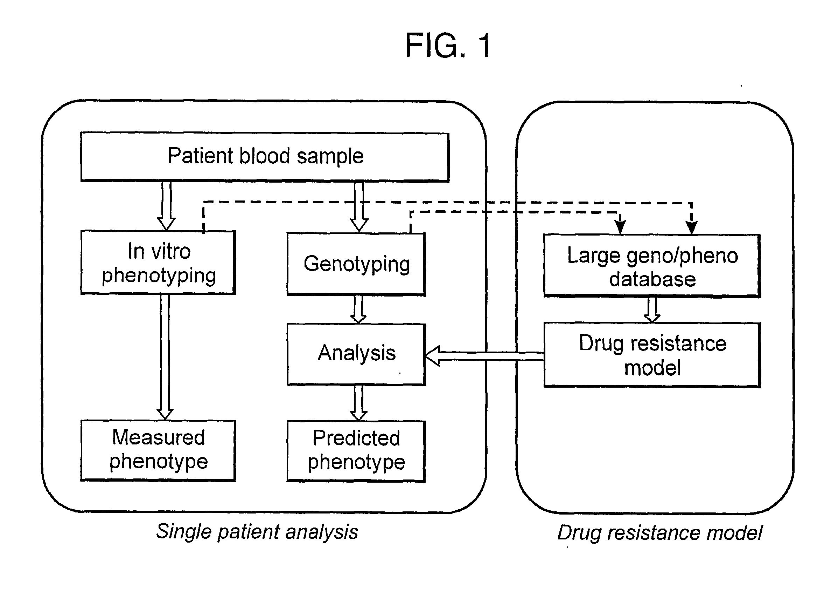

Figure 1: Overview of measured /predicted phenotype handling

Figure 2: Phenotype distribution of ritonavir for matching G/P samples in Virco database

Figure 3: pFR distribution for saquinavir

Figure 4: Distribution of pFR for '48V' mutation on saquinavir

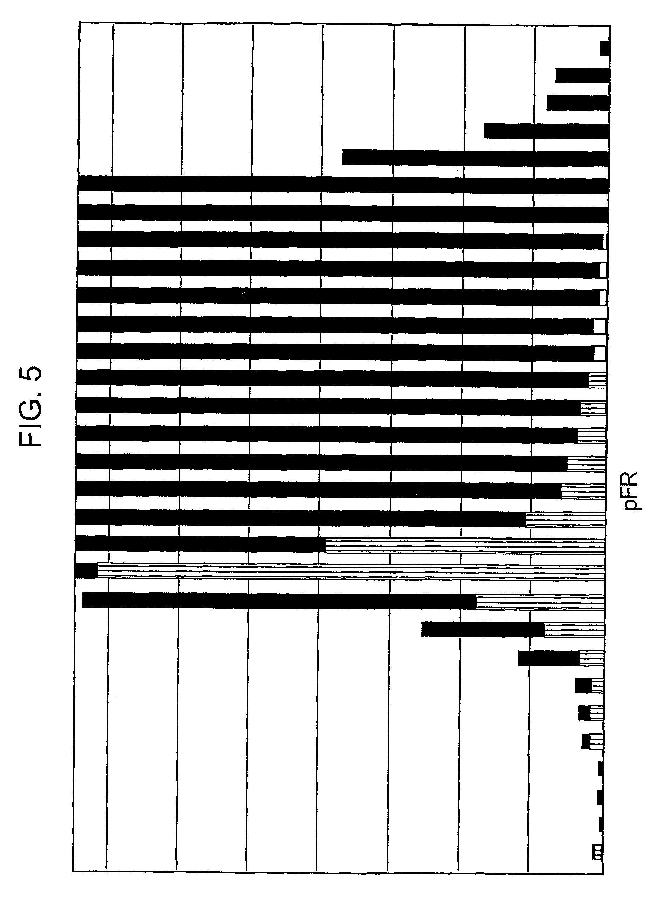

Figure 5: Distribution of pFR for '48V' mutation on saquinavir (expanded)

Figure 6: Removing unwanted correlations

Figure 7: global mutation trajectories

Figure 8: mutation trajectories for 71I

Figure 9: Example of genotypes, mutations relative to HBX2

Figure 10: Example of phenotype analysis for ritonavir

Figure 11: Higher order interaction between mutations 82A and 84V

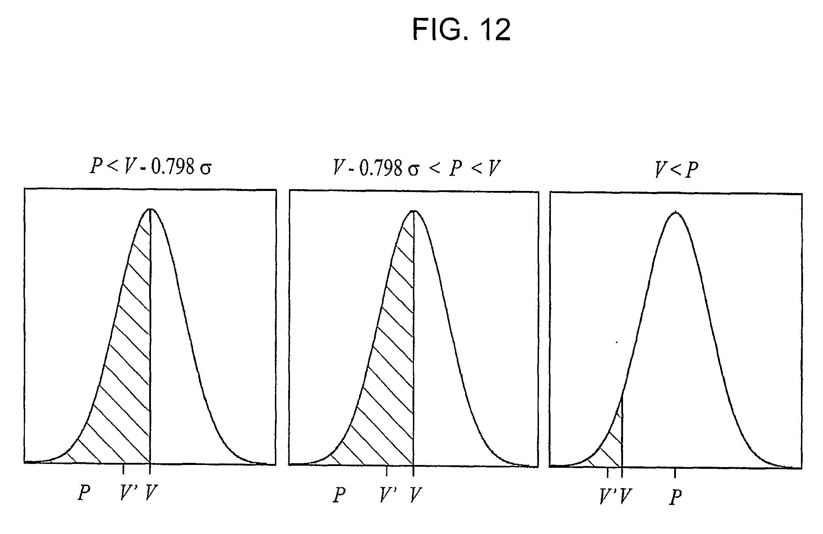

Figure 12: Illustration of iterative procedure for censored values

Figure 13: Linear regression model identifies mutations included in IAS list. Mutations marked with an * are also identified by a regression on a 5% subset of the data

Figure 14: Linear regression model identifies additional mutations previously described in the literature

Figure 15: Predicted versus measured log(FC)

Figure 16: Comparison between linear regression model and decision trees.

Figure 17: Histogram of population left after removing all virus strains during the iterations

Figure 18: Trajectory of mutation 18H

Figure 19: Residues as a function of the measured values

Figure 20: Histogram of the residues as a function of the measured values

Figure 21: Histogram of the residues as a function of the measured values after 6 parameters were taken into account

**EXAMPLES**

**Example 1: Methodology**

**1.1 Introduction**

This exercise involved the generation of a list of key mutations for each of the following drugs: Indinavir, Ritonavir, Saquinavir, Nelfinavir, Amprenavir, Lopinavir, Zidovudine, Didanosine, Zalcitabine, Stavudine, Abacavir, Lamivudine, Tenofovir, Nevirapine, Delavirdine and Efavirenz.
The obtained list of key mutations is derived from a linear regression model using single mutations and couples of mutations as independent variables. The dataset used for this analysis is an export of the Virco dataset at 2003/02/01 from the *vircomining* tables. Table 1 shows the matching geno/pheno counts for each drug *(each* phenotype measurement for a genotype counts as one measurement).

Table 1: matching geno/pheno counts

| Drug | Count | Drug | Count | Drug | Count |
|---|---|---|---|---|---|
| Amprenavir | 29,508 | Lamivudine | 34,395 | Delavirdine | 32,450 |
| Indinavir | 34,445 | Abacavir | 32,744 | Efavirenz | 32,601 |
| Lopinavir | 7,410 | Stavudine | 34,420 | Nevirapine | 34,738 |
| Nelfinavir | 34,470 | Zalcitabine | 34,539 | | |
| Ritonavir | 34,502 | Didanosine | 34,227 | | |
| Saquinavir | 34,543 | Tenofovir | 14,591 | | |
| | | Zidovudine | 33,575 | | |

### 1.2 Dataset

The used dataset consists of a set of matching genotype/phenotype measurements with possible multiple phenotype measurements per genotype. The mutations are defined relative to HXB2 at amino acid level. The phenotypes are presented as *pFR* values, which is equal to - *log (FR),* where *FR* denotes the *Fold Resistance.*

Negative *pFR* values denote resistance and positive values denote hyper-susceptibility. For example, a *pFR* value of -1.0 is equal to 10-fold resistance.

### 1.3 Linear regression

For example, consider the following (artificial) model:

Table 2

| Mutation | Coefficient |
|---|---|
| 84V | -0.46 |
| 50V | -0.92 |
| 54M | -0.64 |
| 88S | 0.63 |
| 90M | -0.16 |
| 46L | -0.19 |
| 46L & 84V | -0.09 |

Consider a virus with following mutations: 3I, 46L, 84V and 90M. This virus will have a *pFR* prediction:

$$pFR = \beta_{46L}.1 + \beta_{84V}.1 + \beta_{90M}.1 + \beta_{46L,84V}.1 = -0.9$$

or almost 8-fold resistance. Note that in the model 46L and 84V are synergistic since their co-occurrence decreases the *pFR* by an extra - 0.09. Note that in the model the mutation 3I shows no resistance coefficient assigned and therefore it is not considered in the *pFR* prediction equation.

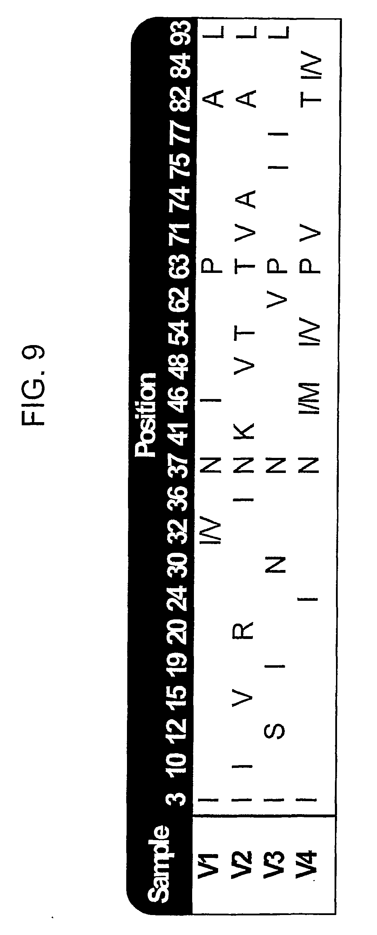

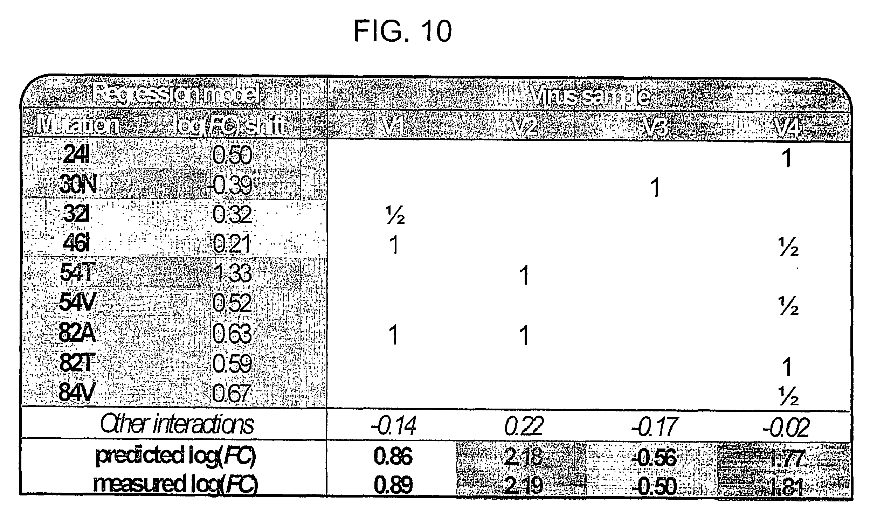

Figure 9 shows an example of four different genotypes (mutations relative to HBX2), whilst Figure 10 shows an example of phenotype analysis for RTV performed according to the method of the invention.

### 1.4 Model creation

Using our facilities, it was computationally infeasible to calculate a full second order model on all possible mutations and second order terms, since the number of terms increases quadratically with the number of mutations considered.

E.g. for APV:

● Total number of occurring mutations and couples of mutations: 19,074
● mutations and couples with each at least 20 measurements: 4,107

In order to reduce the amount of terms, a first order regression was performed from the list of mutations that occur at least 20 times in the dataset. The significant terms from this regression were withheld and the list of these terms -except mutation 3I for some of the PI's, was used to perform a second order regression. A term is called significant if the probability that the real value of the term is 0, is smaller than 0.001. A mutation must occur at least 20 times and the inverse also has to be true: the count of viruses *not* having the mutation 3I or the couple *not* 3I and another mutation should also be at least 20. Taking this into account results in excluding 3I from the regression in practice.In the second order regression only the single mutations and couples of mutations are used that were significant in the first order model.

## 1.5 Example of model creation: Indinavir

A first order regression is performed on the matching geno/pheno dataset (34,445 measurements of which 28,480 unique Virco IDs) for those mutations that occur at least 20 times. The resulting first order model withholds a list of 94 single mutations that are considered significant. This list (except 3I) is used as a starting list for a second order regression. In this second order regression, all the single mutations and all couples of mutations from the list are used as potential terms. The significant terms are withheld by the regression algorithm.

## 1.6 The impact of cross-drug correlation on the significance level of mutations

Correlation between mutations that cause resistance to different drugs, has an impact on the confidence of the coefficient for this mutation. One of the effects is that for non-nucleoside reverse transcriptase inhibitors (NNRTIs) and nucleoside reverse transcriptase inhibitors (NRTIs), some non-relevant mutations for that drug appear as significant (though with a coefficient close to 0), because drug resistance to the drug is correlated with drug resistance to drugs that bind at a different place.

Note that this is only a problem for *interpretation* of the model. For *prediction* of the Fold Resistance, the resulting model remains a good *pFR* predictor.

## 1.7 Effects of second order terms

● Antagonism

Table 3

| Parameter | pFR shift | Count |
|---|---|---|
| ••• | ••• | ••• |
| 82A & 84V | 0.43 | 395 |
| ••• | ••• | ••• |
| 82A | -0.27 | 4845 |
| ••• | ••• | ••• |
| 84V | -0.26 | 3531 |
| ••• | ••• | ••• |

Second order terms can indicate a *synergy* or an *antagonism.* In the example above, the occurrence of either 82A or 84V cause a resistance shift, but the co-occurrence of both mutations almost completely cancels out the effect of both mutations and shifts to susceptible ranges. In case both mutations are present, the net pFR shift is only -0.43, while it is -0.26 or -0.27 if only one of the mutations are present. This is an example of strong antagonism.

● Synergy

Table 4

| Parameter | pFR shift | Count |
| --- | --- | --- |
| ••• | ••• | ••• |
| 24I | -0.22 | 1022 |
| ••• | ••• | ••• |
| 24I & 73S | -0.48 | 30 |
| ••• | ••• | ••• |
| 73S | -0.20 | 2216 |
| ••• | ••• | ••• |

In this example, 24I and 73S both cause a resistance shift, but their co-occurrence causes a strong extra shift towards resistance. When only one of the mutations is present, the *pFR* shift is -0.20 or -0.22, but the presence of both mutations causes a *pFR* shift of -0.48. 24I and 73S are thus strongly synergistic in this example.

● Enhancement

Table 5

| Parameter | pFR shift | Count |
| --- | --- | --- |
| ••• | ••• | ••• |
| 32I | 0 | 821 |
| ••• | ••• | ••• |
| 32I & 82A | -0.26 | 516 |
| ••• | ••• | ••• |
| 82A | -0.27 | 4845 |
| ••• | ••• | ••• |

32I by itself does not contribute to resistance, but it increases the resistance for an 82A mutation. 32I enhances the effect of the 82A mutation.

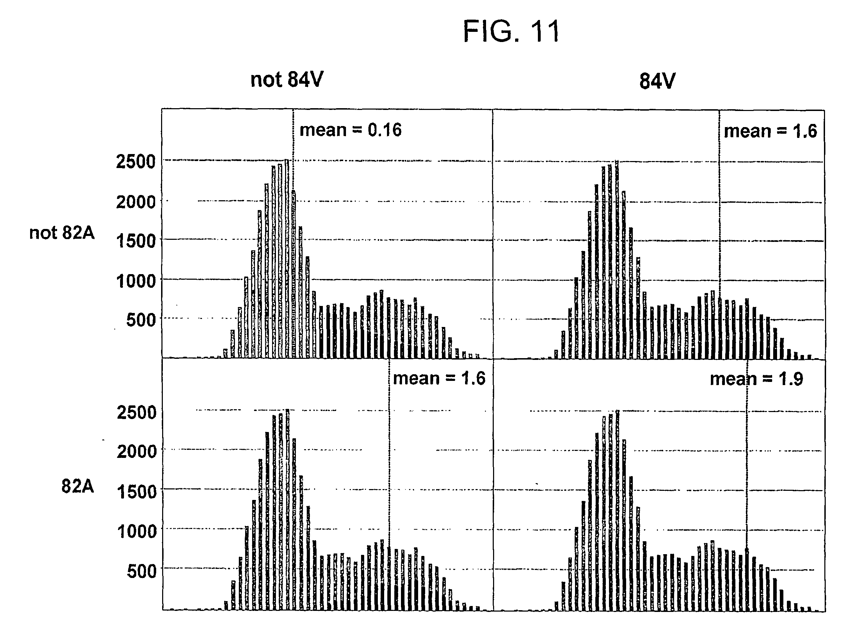

An example of the effects of higher order interactions is shown in Figure 11.

### 1.8 Highly correlated mutations

Highly correlated mutations (i.e. mutations that almost always co-occur in a strain) can affect the results of a regression analysis For example, when one of these 2 mutations has an effect on resistance and the other mutation does not (this mutation might for example be highly correlated with the resistance mutation because it affects the replication rate of the virus), the effect can be assigned to either one of the mutations. Unless this is compensated for, the regression model will assign the effect to that mutation that reduces the prediction error the most, which might not always be the mutation that is biologically responsible for the effect. Due to the correlation, it would otherwise not be possible to distinguish between these mutations.

Another effect that occurs due to correlation is when a mutation is highly correlated with a pair of mutations in which the first mutation is present.

Table 6

| Parameter | pFR shift | Count |
|---|---|---|
| ••• | ••• | |
| 58N | -1.47 | 108 |
| ••• | ••• | |
| 58N & 77L | 1.16 | 106 |
| ••• | ••• | |
| 77L | 0 | 471 |
| ••• | ••• | |

In the above example, 108 samples have a 58N mutation and out of these, 106 samples also have a 77L mutation. The effect of a pure 58N mutation can only be derived from the samples that have 58N and do not have 77L, which leads to higher uncertainty on the estimated *pFR* shift of the 58N mutation. The couple-term '58N & 77L' will compensate for a too low estimation of 58N by having a too high estimation for its *pFR* shift.

Techniques are provided in the description to deal with these effects. The algorithm developed by the inventors tracks the change in pFR as the effects of individual mutations or combinations of mutations are removed from the dataset.

A comparison of the change in the global average pFR with the change in the average pFR for selected mutations with increasing iterations of the algorithm is shown in Figure 7. Figure 8 shows an example, where the average pFR for 71I (unwanted correlation) jumps up as a result of removing from the dataset virus strains that have "71I & 84V" and "48V" mutations.

### Example 2: Illustration of a stepwise regression with amprenavir

In a dataset of 31,292 matching genotypes and phenotypes for amprenavir; a stepwise first order regression was performed, and the first 11 iterations are shown here. The inventors selected as p-values:

p-value entry = 0.001
p-value stay = 0.5

After the first iteration all the variables with mutations found more than 20 times were selected. In the next step the p-value was calculated, and then the algorithm picked the variable with the lowest p-value. In this case it was P084V, and then a model was built with this variable (84V). The model is shown in
Table 7.
*Variable P084_V Entered: R-Square = 0.2477 and C(p) = 43445.43*

Table 7

| Analysis of Variance | | | | | |
|---|---|---|---|---|---|
| Source | DF | Sum of Squares | Mean Square | F Value | Pr > F |
| Model | 1 | 2042.55324 | 2042.55324 | 10301.6 | <.0001 |
| Error | 31289 | 6203.83650 | 0.19828 | | |
| Corrected Total | 31290 | 8246.38974 | | | |
| | | | | | |
| Variable | Parameter Estimate | Standard Error | Type II SS | F Value | Pr > F |
| Intercept | -0.00318 | 0.00269 | 0.27810 | 1.40 | 0.2363 |
| P084_V | -0.81754 | 0.00805 | 2042.55324 | 10301.6 | <.0001 |

In the next iteration, we repeated the previous process except that the influence of 84V is now removed. In this second run, the mutation with the lowest p-values was P082A. The model is shown in
Table 8 below.

*Variable P082_A Entered: R-Square = 0.3538 and C(p) = 32908.89*

Table 8

| Analysis of Variance | | | | | |
|---|---|---|---|---|---|
| Source | DF | Sum of Squares | Mean Square | F Value | Pr>F |
| Model | 2 | 2917.39937 | 1458.69968 | 8564.44 | <.0001 |
| Error | 31288 | 5328.99038 | 0.17032 | | |
| Corrected Total | 31290 | 8246.38974 | | | |
| | | | | | |
| Variable | Parameter Estimate | Standard Error | Type II SS | F Value | Pr > F |
| Intercept | 0.07033 | 0.00269 | 116.28598 | 682.75 | <.0001 |
| P082_A | -0.47905 | 0.00668 | 874.84612 | 5136.47 | <.0001 |
| P084_V | -0.82827 | 0.00747 | 2095.67091 | 12304.3 | <.0001 |

In the next iteration, we repeated the previous process except that the influence of 84V and 82A is now removed. In this third run, the mutation with the lowest p-values was P090M.

It may happen that after some iterations, a mutation which was found first significant is not significant anymore, and as such it is removed from the model.

The resistance coefficient is adjusted after every iteration because of the addition of a new variable to the regression. E.g. for P84V, the resistance coefficient β changes from -0.81754 to -0.82827.

The following iterations were done in the same way. The results obtained are here below enclosed.

*Variable P090_M Entered: R-Square = 0.4245 and C(p) = 25885. 06*

Table 9

| Analysis of Variance | | | | | |
|---|---|---|---|---|---|
| Source | DF | Sum of Squares | Mean Square | F Value | Pr > F |
| Model | 3 | 3500.64130 | 1166.88043 | 7692.82 | <.0001 |
| Error | 31287 | 4745.74844 | 0.15168 | | |
| Corrected Total | 31290 | 8246.38974 | | | |
| | | | | | |
| Variable | Parameter Estimate | Standard Error | Type II SS | F Value | Pr>F |
| Intercept | 0.13680 | 0.00276 | 373.40964 | 2461.75 | <.0001 |
| P082 A | -0.40431 | 0.00642 | 601.20353 | 3963.52 | <.0001 |
| P084_V | -0.64830 | 0.00762 | 1097.72370 | 7236.89 | <.0001 |
| P090_M | -0.33549 | 0.00541 | 583.24194 | 3845.10 | <.0001 |

*Variable P033_F Entered: R-Square = 0.4729 and C(p) = 21082.65*

Table 10

| Analysis of Variance | | | | | |
|---|---|---|---|---|---|
| Source | DF | Sum of Squares | Mean Square | F Value | Pr > F |
| Model | 4 | 3899.47454 | 974.86863 | 7016.41 | <.0001 |
| Error | 31286 | 4346.91521 | 0.13894 | | |
| Corrected Total | 31290 | 8246.38974 | | | |
| | | | | | |

(continued)

| Variable | Parameter Estimate | Standard Error | Type II SS | F Value | Pr > F |
|---|---|---|---|---|---|
| Intercept | 0.14140 | 0.00264 | 398.56350 | 2868.58 | <.0001 |
| P033_F | -0.60263 | 0.01125 | 398.83323 | 2870.52 | <.0001 |
| P082_A | -0.35755 | 0.00621 | 460.87758 | 3317.07 | <.0001 |
| P084_V | -0.60772 | 0.00733 | 954.28785 | 6868.28 | <.0001 |
| P090_M | -0.30702 | 0.00521 | 483.38315 | 3479.05 | <.0001 |

*Variable P046_I Entered: R-Square = 0.5110 and C(p) =17296.94*

Table 11

| Analysis of Variance | | | | | |
|---|---|---|---|---|---|
| Source | DF | Sum of Squares | Mean Square | F Value | Pr>F |
| Model | 5 | 4213.90735 | 842.78147 | 6538.51 | <.0001 |
| Error | 31285 | 4032.48240 | 0.12890 | | |
| Corrected Total | 31290 | 8246.38974 | | | |
| Variable | Parameter Estimate | Standard Error | Type II SS | F Value | Pr > F |
| Intercept | 0.15801 | 0.00257 | 489.12701 | 3794.77 | <.0001 |
| P033_F | -0.59310 | 0.01084 | 386.19296 | 2996.18 | <.0001 |
| P046_I | -0.32298 | 0.00654 | 314.43281 | 2439.45 | <.0001 |
| P082_A | -0.32721 | 0.00601 | 381.95629 | 2963.31 | <.0001 |
| P084_V | -0.53666 | 0.00721 | 714.53640 | 5543.55 | <.0001 |
| P090_M | -0.25737 | 0.00511 | 326.53882 | 2533.37 | <.0001 |

*Variable P047_V Entered: R-Square = 0.5287 and C(p) =15544.70*

Table 12

| Analysis of Variance | | | | | |
|---|---|---|---|---|---|
| Source | DF | Sum of Squares | Mean Square | F Value | Pr > F |
| Model | 6 | 4359.53390 | 726.58898 | 5848.07 | <.0001 |
| Error | 31284 | 3886.85584 | 0.12424 | | |
| Corrected Total | 31290 | 8246.38974 | | | |
| | | | | | |
| Variable | Parameter Estimate | Standard Error | Type II SS | F Value | Pr > F |
| Intercept | 0.15941 | 0.00252 | 497.68905 | 4005.73 | <.0001 |
| P033_F | -0.55617 | 0.01069 | 336.13339 | 2705.42 | <.0001 |
| P046_I | -0.27816 | 0.00655 | 223.90478 | 1802.13 | <.0001 |
| P047_V | -0.63671 | 0.01860 | 145.62656 | 1172.10 | <.0001 |
| P082_A | -0.32763 | 0.00590 | 382.93722 | 3082.13 | <.0001 |
| P084_V | -0.54696 | 0.00708 | 740.88446 | 5963.13 | <.0001 |
| P090_M | -0.25573 | 0.00502 | 322.35883 | 2594.56 | <.0001 |

*Variable P046_L Entered: R-Square = 0.5409 and C(p) = 14326.32*

Table 13

| Analysis of Variance | | | | | |
|---|---|---|---|---|---|
| Source | DF | Sum of Squares | Mean Square | F Value | |
| Model | 7 | 4460.84208 | 637.26315 | 5266.21 | <.0001 |
| Error | 31283 | 3785.54767 | 0.12101 | | |
| Corrected Total | 31290 | 8246.38974 | | | |
| | | | | | |
| Variable | Parameter Estimate | Standard Error | Type II SS | F Value | Pr>F |
| Intercept | 0.16438 | 0.00249 | 526.67853 | 4352.36 | <.0001 |
| P033_F | -0.54921 | 0.01056 | 327.61200 | 2707.32 | <.0001 |
| P046_I | -0.31345 | 0.00658 | 274.55948 | 2268.90 | <.0001 |
| P046_L | -0.27769 | 0.00960 | 101.30817 | 837.19 | <.0001 |
| P047_V | -0.63940 | 0.01835 | 146.85706 | 1213.60 | <.0001 |
| P082_A | -0.26575 | 0.00620 | 222.00011 | 1834.56 | <.0001 |
| P084_V | -0.52972 | 0.00702 | 689.88420 | 5701.06 | <.0001 |
| P090_M | -0.24267 | 0.00498 | 287.89278 | 2379.09 | <.0001 |

*Variable P050_V Entered: R-Square = 0.5534 and C(p) =13094.82*

Table 14

| Analysis of Variance | | | | | |
|---|---|---|---|---|---|
| Source | DF | Sum of Squares | Mean Square | F Value | Pr > F |
| Model | 8 | 4563.24026 | 570.40503 | 4844.61 | <.0001 |
| Error | 31282 | 3683.14948 | 0.11774 | | |
| Corrected Total | 31290 | 8246.38974 | | | |
| | | | | | |
| Variable | Parameter Estimate | Standard Error | Type II SS | F Value | Pr>F |
| Intercept | 0.16629 | 0.00246 | 538.66106 | 4575.00 | <.0001 |
| P033_F | -0.51815 | 0.01046 | 288.64295 | 2451.52 | <.0001 |
| P046_I | -0.29956 | 0.00651 | 249.44262 | 2118.58 | <.0001 |
| P046_L | -0.27934 | 0.00947 | 102.51249 | 870.67 | <.0001 |
| P047_V | -0.64754 | 0.01811 | 150.58308 | 1278.94 | <.0001 |
| P050_V | -0.82797 | 0.02808 | 102.39819 | 869.70 | <.0001 |
| P082_A | -0.25944 | 0.00612 | 211.32860 | 1794.87 | <.0001 |
| P084_V | -0.53915 | 0.00693 | 713.14511 | 6056.94 | <.0001 |
| P090_M | -0.24415 | 0.00491 | 291.39382 | 2474.89 | <.0001 |

*Variable P054_M Entered: R-Square = 0.5643 and C(p) = 12005.72*

Table 15

| Analysis of Variance | | | | | |
|---|---|---|---|---|---|
| Source | DF | Sum of Squares | Mean Square | F Value | Pr > F |
| Model | 9 | 4653.81644 | 517.09072 | 4502.38 | <.0001 |
| Error | 31281 | 3592.57330 | 0.11485 | | |
| Corrected Total | 31290 | 8246.38974 | | | |
| | | | | | |
| Variable | Parameter Estimate | Standard Error | Type II SS | F Value | Pr > F |
| Intercept | 0.16633 | 0.00243 | 538.92765 | 4692.51 | <.0001 |
| P033_F | -0.46793 | 0.01049 | 228.56387 | 1990.14 | <.0001 |
| P046_I | -0.30134 | 0.00643 | 252.40077 | 2197.69 | <.0001 |
| P046_L | -0.28508 | 0.00935 | 106.71653 | 929.19 | <.0001 |
| P047_V | -0.55613 | 0.01818 | 107.50855 | 936.09 | <.0001 |
| P050_V | -0.84757 | 0.02774 | 107.23423 | 933.70 | <.0001 |
| P054_M | -0.57119 | 0.02034 | 90.57618 | 788.66 | <.0001 |
| P082_A | -0.26050 | 0.00605 | 213.05431 | 1855.09 | <.0001 |
| P084_V | -0.53337 | 0.00685 | 697.31870 | 6071.64 | <.0001 |
| P090_M | -0.23454 | 0.00486 | 267.57375 | 2329.80 | <.0001 |

*Variable P054_L Entered: R-Square = 0.5740 and C(p) =11047.41*

Table 16

| Analysis of Variance | | | | | |
|---|---|---|---|---|---|
| Source | DF | Sum of Squares | Mean Square | F Value | Pr > F |
| Model | 10 | 4733.53615 | 473.35362 | 4214.95 | <.0001 |
| Error | 31280 | 3512.85359 | 0.11230 | | |
| Corrected Total | 31290 | 8246.38974 | | | |
| | | | | | |
| Variable | Parameter Estimate | Standard Error | Type II SS | F Value | Pr > F |
| Intercept | 0.16756 | 0.00240 | 546.67520 | 4867.84 | <.0001 |
| P033_F | -0.40056 | 0.01068 | 158.09630 | 1407.76 | <.0001 |
| P046_I | -0.30750 | 0.00636 | 262.46469 | 2337.10 | <.0001 |
| P046_L | -0.28311 | 0.00925 | 105.23686 | 937.08 | <.0001 |
| P047_V | -0.52353 | 0.01802 | 94.83277 | 844.43 | <.0001 |
| P050_V | -0.87024 | 0.02744 | 112.94107 | 1005.68 | <.0001 |
| P054_L | -0.456111 | 0.01712 | 79.71971 | 709.86 | <.0001 |
| P054 M | -0.61432 | 0.02018 | 104.09593 | 926.92 | <.0001 |
| P082_A | -0.26465 | 0.00598 | 219.74203 | 1956.68 | <.0001 |
| P084_V | -0.51838 | 0.00679 | 654.14626 | 5824.81 | <.0001 |
| P090_M | -0.22560 | 0.00482 | 246.35798 | 2193.68 | <.0001 |

*Variable P088_S Entered: R-Square = 0.5834 and C(p) =10116.87*

Table 17

| Analysis of Variance | | | | | |
|---|---|---|---|---|---|
| Source | DF | Sum of Squares | Mean Square | F Value | Pr > F |
| Model | 11 | 4810.94944 | 437.35904 3 | 982.07 | <.0001 |
| Error | 31279 | 3435.44030 | 0.10983 | | |
| Corrected Total | 31290 | 8246.38974 | | | |
| | | | | | |
| Variable | Parameter Estimate | Standard Error | Type II SS | F Value | Pr > F |
| Intercept | 0.16239 | 0.00238 | 510.05266 | 4643.93 | <.0001 |
| P033_F | -0.39933 | 0.01056 | 157.11889 | 1430.54 | <.0001 |
| P046_I | -0.32619 | 0.00633 | 291.69184 | 2655.80 | <.0001 |
| P046_L | -0.29089 | 0.00915 | 110.98928 | 1010.54 | <.0001 |
| P047_V | -0.50941 | 0.01782 | 89.70713 | 816.77 | <.0001 |
| P050_V | -0.86163 | 0.02714 | 110.70009 | 1007.90 | <.0001 |
| P054_L | -0.46062 | 0.01693 | 81.29492 | 740.17 | <.0001 |
| P054_M | -0.61674 | 0.01995 | 104.91704 | 955.25 | <.0001 |
| P082_A | -0.25645 | 0.00592 | 205.77424 | 1873.53 | <.0001 |
| P084_V | -0.51212 | 0.00672 | 637.66342 | 5805.80 | <.0001 |
| P088_S | 0.57942 | 0.02182 | 77.41329 | 704.83 | <.0001 |
| P090_M | -0.22099 | 0.00477 | 236.06351 | 2149.31 | <.0001 |

**Example 3: Dealing with censored values**

In this example, the method developed by the inventors to deal with censored values was applied for the drug amprenavir. Firstly, a linear regression model without censored values was calculated, see the values 'APV_pFR' for iteration 0 in the Tables 18-21 below. The phenotypic measured valueis < -2.083062017 for Virus strain 1, for the first iteration, the value $V_0$ is equal to the measured phenotype value APV_pFR, but the censor is considered as '='. This was followed by iteration nr. 1. Once the values were obtained from iteration 1, the inventors compared the prediction value P, e.g. for Virus strain 1, -2.362076895 with the phenotypic measured value <-2.083062017. For virus strains 1, 2 and 3 below, the measured values have a censor '<'. For virus strains 4, the measured value has a censor '>'. The scenarios established by the inventors, when the case was '<'-censor, and when the case was '>' -censor, were applied. New linear regression models were calculated and for the censored values in the linear regression model, either the data-points from the training set were removed, as for virus strains 1, 2 and for virus strain 4 (iteration 2 and 3 only), or those data-points were used instead of the censored phenotypes measurement, as illustrated for virus strain 3 and for virus strain 4 (only iteration 1). The procedure was reiterated until the prediction converged.

Virus strain 1: CASE '<' and P < V - 0.798 $\sigma$
V - 0.798 $\sigma$ = -2.282562017

Table 18

| ITER | APV_censor | APV_pFR | Prediction P | value |
|---|---|---|---|---|
| 0 | < | -2.083062017 | | -2.083062017 |
| 1 | < | -2.083062017 | -2.362076895 | |
| 2 | < | -2.083062017 | -2.537358188 | |
| 3 | < | -2.083062017 | -2.550836421 | |

Virus strain 2: CASE '<' and P < V - 0.798 σ

V - 0.798 σ = -2.108607374

Table 19

| ITER | APV_censor | APV pFR | Prediction P | value |
|---|---|---|---|---|
| 0 | < | -1.909107374 | | -1.909107374 |
| 1 | < | -1.909107374 | -2.608207782 | |
| 2 | < | -1.909107374 | -2.743936505 | |
| 3 | < | -1.909107374 | -2.748739259 | |

Virus strain 3: CASE '<' and V <= P

V - 0.798 σ = -2.156216334

Table 20

| ITER | APV_censor | APV _pFR | Prediction P | value |
|---|---|---|---|---|
| 0 | < | -1.956716334 | | -1.956716334 |
| 1 | < | -1.956716334 | -1.343253355 | -2.016673816 |
| 2 | < | -1.956716334 | -1.401494738 | -2.021216328 |
| 3 | < | -1.956716334 | -1.405764249 | -2.02157333 |

Virus strain 4: CASE '>' and V < P <= V + 0.798 σ

V + 0.798 σ = 0.714928642

Table 21

| ITER | APV_censor | APV_pFR | Prediction P | value |
|---|---|---|---|---|
| 0 | > | 0.515428642 | | 0.515428642 |
| 1 | > | 0.515428642 | 0.671585565 | 0.714928642 |
| 2 | > | 0.515428642 | 0.759236384 | |
| 3 | > | 0.515428642 | 0.770724812 | |

**Example 4: Mutations trajectories**

A methodology for removing unwanted correlations involved an algorithm developed by the inventors in which the change in pFR was tracked as the effects of individual mutations or combinations of mutations were removed from the dataset. The effect of each mutation or combination of mutations was separated out. The methodology followed mutation trajectories towards the global average as the effects of individual mutations or combinations of mutations were removed.

In a first step, the average pFR was calculated for all mutations with a sufficient count in the database to be significant, i.e. >20. In the Table below the first and last 10 mutations of a list of 368 mutations is shown with the corresponding calculated average pFR.

Table 22

| Label | Count | Average pFR |
|---|---|---|
| 54M | 239 | -1.35421631044816 |
| 50V | 129 | -1.28868671545329 |
| 47V | 281 | -1.27082426054035 |
| 84A | 21 | -1.23536347179756 |

(continued)

| Label | Count | Average pFR |
|---|---|---|
| 76V | 187 | -1.20328089469875 |
| 89V | 269 | -1.19273200093637 |
| 91S | 36 | -1.15103609689224 |
| 33M | 22 | -1.12268052165089 |
| 84C | 21 | -1.11529918764429 |
| 54L | 327 | -1.09959239280592 |
| ... | | |
| 18L | 65 | 0.12143300387582 |
| 69K | 1387 | 0.121561573218861 |
| 89M | 1261 | 0.134344491996282 |
| 33V | 406 | 0.140725379487564 |
| 17E | 86 | 0.154734960149477 |
| 63I | 20 | 0.159585638802379 |
| 62M | 22 | 0.184594303565984 |
| 19S | 21 | 0.202798973752203 |
| 15L | 51 | 0.213932584381636 |
| 88S | 204 | 0.494820688128991 |

The extremes were determined, and the mutation with the pFR furthest away from the global average was selected. In Table 22, the mutation selected was 54M.

Following, all virus strains that had the selected mutation were removed, in total it amounted to 283 virus strains: from 26738 to 26455. A new Table 23 of average pFR was generated for the remaining mutations. The list below shows the first and last 10 mutations of the obtained results.

Table 23

| New table of average per mutation: | | |
|---|---|---|
| Label | Count | Average pFR |
| 50V | 129 | -1.28868671545329 |
| 84A | 21 | -1.23536347179756 |
| 76V | 168 | -1.13676536523909 |
| 47V | 215 | -1.12849822159876 |
| 91S | 35 | -1.12648669210251 |
| 33M | 22 | -1.12268052165089 |
| 84C | 21 | -1.11529918764429 |
| 54L | 327 | -1.09959239280592 |
| 89V | 213 | -1.05990043312845 |
| 33F | 852 | -0.988257531387902 |
| ... | | |
| 18L | 65 | 0.12143300387582 |
| 69K | 1384 | 0.125380073379929 |

(continued)

| New table of average per mutation: | | |
| --- | --- | --- |
| Label | Count | Average pFR |
| 89M | 1256 | 0.141136938991525 |
| 33V | 404 | 0.147380396015872 |
| 17E | 86 | 0.154734960149477 |
| 63I | 20 | 0.159585638802379 |
| 62M | 22 | 0.184594303565984 |
| 19S | 21 | 0.202798973752203 |
| 15L | 51 | 0.213932584381636 |
| 88S | 202 | 0.494762597743788 |

The previous steps were reiterated. In Table 24 there is listed the mutations which were selected at different iteration counts.

Table 24

| Count | Mutation selected |
| --- | --- |
| 26738 | 54M |
| 26455 | 50V |
| 26296 | 84A |
| 26272 | 91S |
| 26220 | 47V |
| 25947 | 76V |
| 25762 | 54L |
| 25365 | 22V |
| 25260 | 33F |
| 24545 | 32I |
| 24183 | 84V |
| 21784 | 54S |
| 21728 | 82F |
| 21577 | 54T |
| 21427 | 24F |
| 21397 | 24I |
| 20894 | 73T |
| 20702 | 73C |
| 20604 | 55R |
| 20324 | 95F |
| 20194 | 10R |
| 20122 | 54A |
| 20062 | 82C |
| 20039 | 88S |

(continued)

| Count | Mutation selected |
|-------|-------------------|
| 19818 | 46L |
| 19247 | 23I |
| 19190 | 58E |
| 18909 | 67F |
| 18877 | 73S |
| 18105 | 53L |
| 17920 | 10F |
| 17568 | 82A |
| 16637 | 46I |
| 16215 | 54V |
| 16028 | 35G |
| 15996 | 90M |

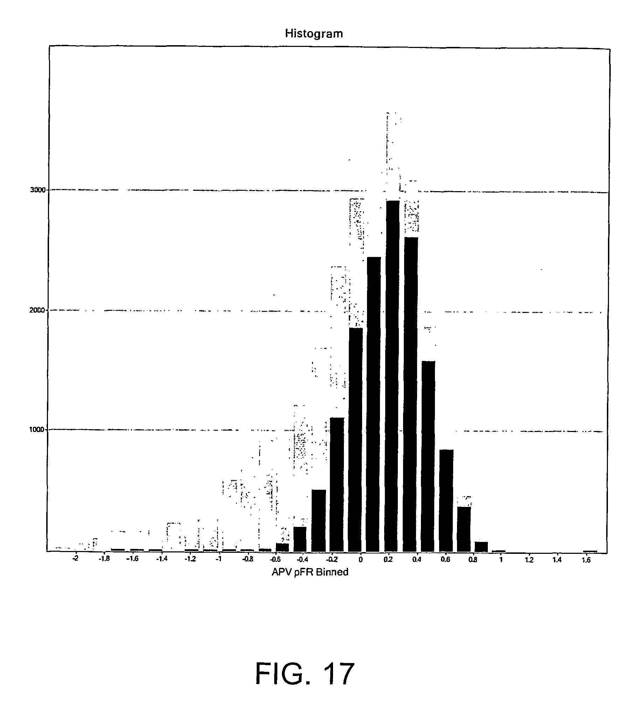

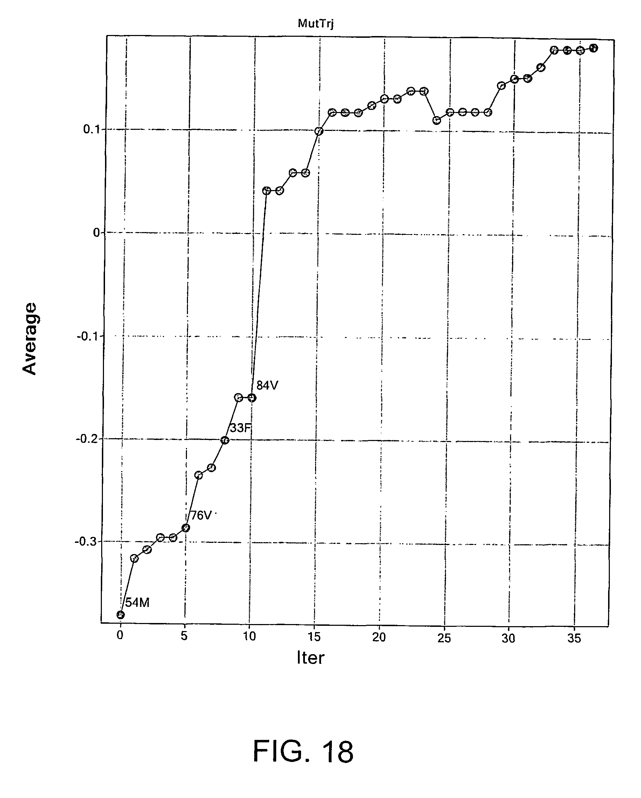

The procedure was stopped when the last selected mutation had an average pFR that approximated to the global average. In Figure 17, a histogram of the population left after removing all virus strains during the iterations is shown. In Figure 18, the trajectory of mutation 18H, which has no significant phenotype, demonstrates the underlying cause that a virus strain is resistant due to other mutations than 18H (54M, 76V, 33F, 84V).

**Example 5: Highly correlated mutations**

A methodology for removing unwanted correlations proceeded as follows. In a first stage, the correlation coefficient between all mutations (with a sufficient count in the database, i.e. >20) and the pFR was calculated. In Table 25 below there is listed the first and last 10 coefficients of a list of 202 coefficients that were calculated.

Table 25

| NAME | correlation with APV pFR |
|------|--------------------------|
| P084_V | -0.51430323209603 |
| P090_M | -0.510166810424169 |
| P010_I | -0.43843660921923 |
| P046_I | -0.430221405121849 |
| P071_V | -0.400337497273138 |
| P033_F | -0.385947043862637 |
| P082*_A | -0.343946685713202 |
| P054_V | -0.339269018202483 |
| P032_I | -0.275105400287351 |
| P010_F | -0.263412991920486 |
| ... | |
| P065 D | 0.044821230615045 |
| P012_A | 0.0476139542192145 |
| P014_R | 0.0485859961407927 |
| P033_V | 0.0571605219086003 |
| P041_K | 0.0713497764580897 |

(continued)

| NAME | correlation with APV pFR |
|------|--------------------------|
| P063_S | 0.071416853283617 |
| P030_N | 0.080199177466301 |
| P089_M | 0.0904859904655573 |
| P069_K | 0.0926109439533826 |
| P088_S | 0.101920449277774 |

Consequently, the extremes were determined (maximum, minimum), and the mutation with the highest (absolute value of) correlation coefficient was selected. In this case was P084_V.

In the following step, a linear model for the pFR with the selected mutation(s) (from step 2, all previous iterations) was calculated. The predicted model obtained was Predicted pFR = -0.844 * $M_{84}$

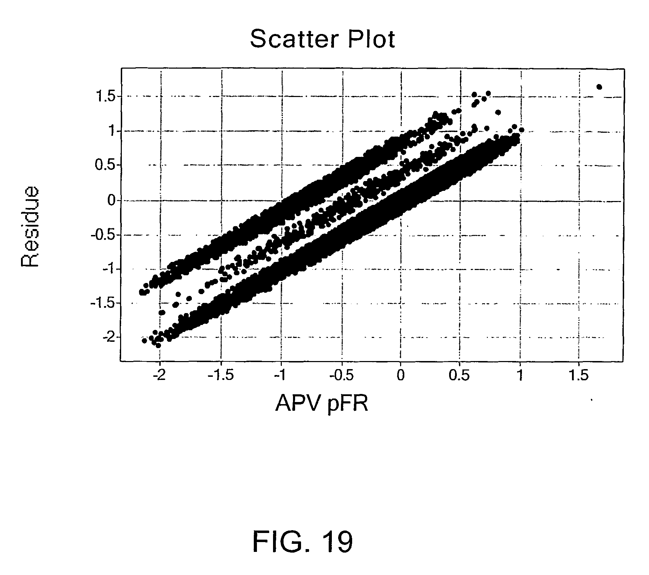

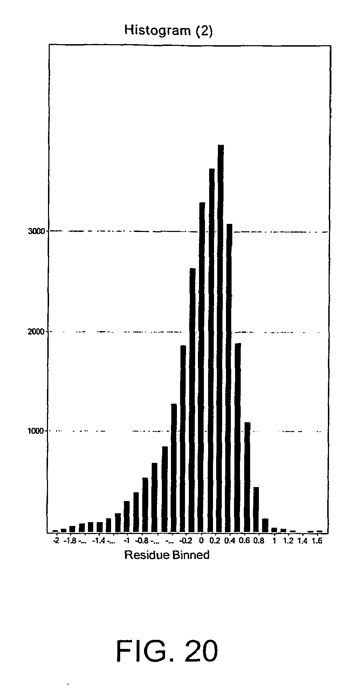

Following, the residue was taken (pFR minus the predicted value from the model). In Figure 19 a graph of the residues as a function of the measured values is shown. In Figure 20, the same graph is represented in the form of histograms where the distribution of the residue may be observed.

Then, the correlation coefficient between all mutations with a sufficient count in the database and the residue was calculated. Results of the first and last 10 variable are shown here below. It will be observed that the order of the mutations had changed because the influence of mutations P084_V had been removed.

Table 26

| NAME | Correlation with Residue |
|------|--------------------------|
| P082_A | -0.402088216120049 |
| P090_M | -0.373018037350597 |
| P033_F | -0.347337204147266 |
| P032_I | -0.325689507827784 |
| P046_I | -0.324078296352946 |
| P010_I | -0.321343698855709 |
| P054_V | -0.311246000758214 |
| P071_V | -0.290388889036061 |
| P047_V | -0.272571613897309 |
| P046_L | -0.234148912637279 |
| ... | |
| P012_S | 0.0358242687063381 |
| P014_R | 0.0401577992704191 |
| P012_A | 0.0442436406740132 |
| P063_S | 0.0484345347109348 |
| P033_V | 0.0537012592855092 |
| P030_N | 0.0548222080117112 |
| P041_K | 0.0659977808671604 |
| P089_M | 0.078693680117922 |
| P069_K | 0.0835173137603877 |
| P088_S | 0.110781153455097 |

Consequently, the extremes were determined again, and the mutation with the highest absolute value of the correlation coefficient was selected. The mutation selected now was P082_A.

A new linear model for the pFR with the selected mutation P082_A, was calculated. *pFR* = -0.782*M84 + -0.435*M82

After 6 iterations, the following resistance coefficients for a selected group of mutations was obtained.

Table 27

| Parameter | β |
|---|---|
| P084_V | -0.512345233450494 |
| P082_A | -0.247464800298171 |
| P090 M | -0.156011849225004 |
| P033_F | -0.532092050020052 |
| P046 I | -0.205719880528639 |
| P047_V | -0.656374746817602 |

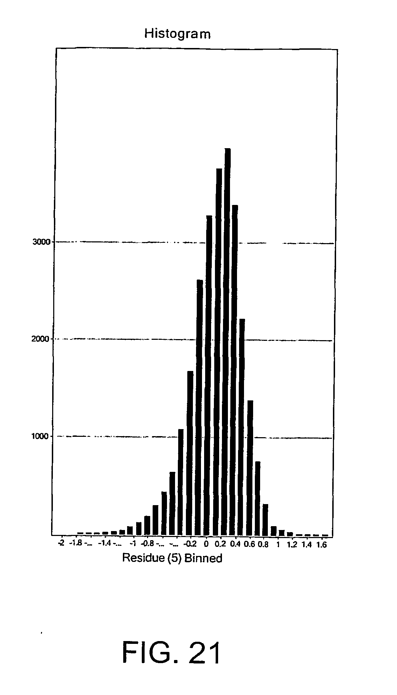

In Figure 21, a graph is represented in the form of histograms showing the residues after the 6 parameters were taken into account.

The reiterative procedure continued until the last selected mutation had a correlation coefficient that approximated to zero.

**Example 6: Results from linear regression modeling**

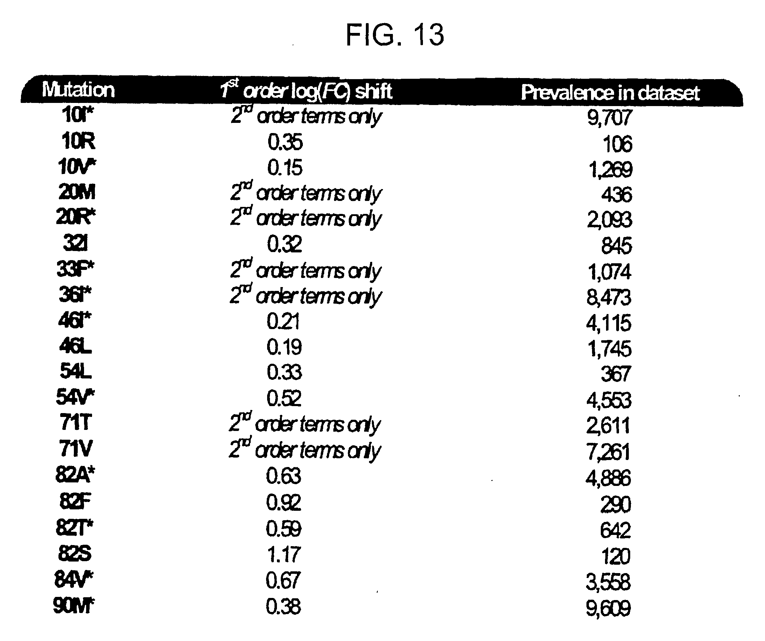

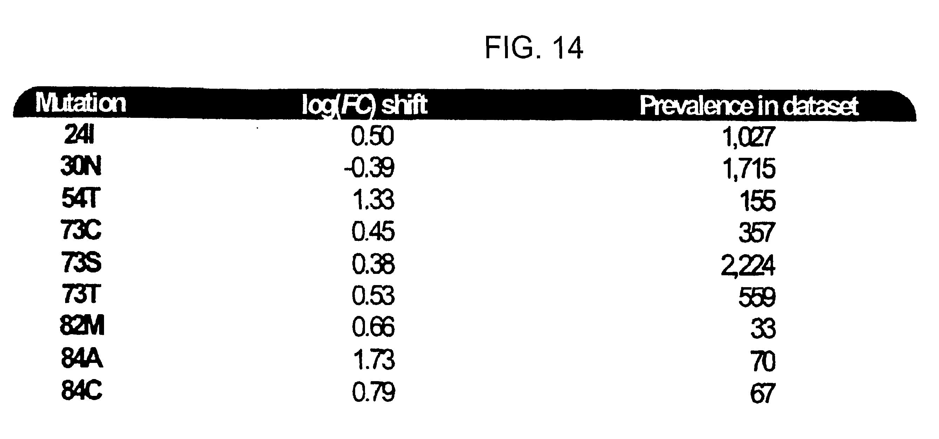

In an initial test, genotypes and corresponding phenotypes determined for ritonavir (RTV) for 28,540 HIV-1 clinical isolates were used. The linear regression analysis identified 20/22 RTV resistance-associated mutations described in the IAS mutation list (all except 10F and 771) (see Figure 13). Additional mutations whose effect on RTV susceptibility had been previously described (e.g. 73S/T/C, 84A/C and 88D) were also identified (Figure 14). Overall, 53 single mutations and 96 pairs of mutations were identified as having significant effect on susceptibility to RTV.

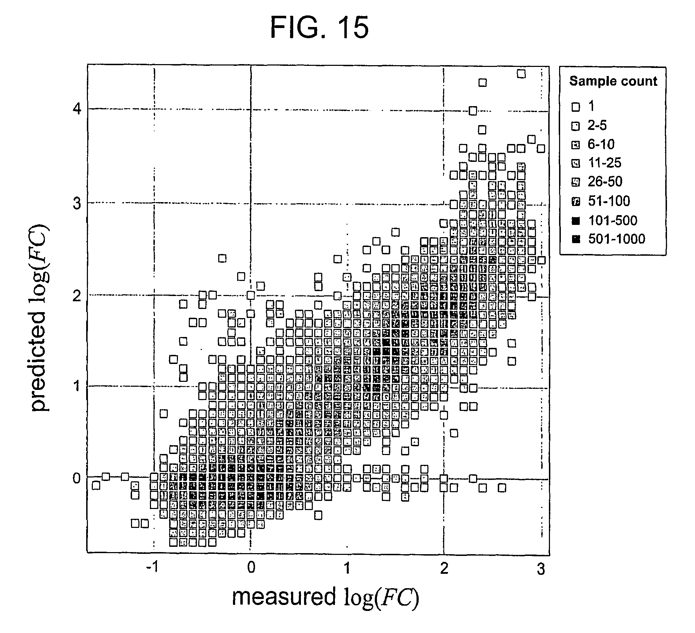

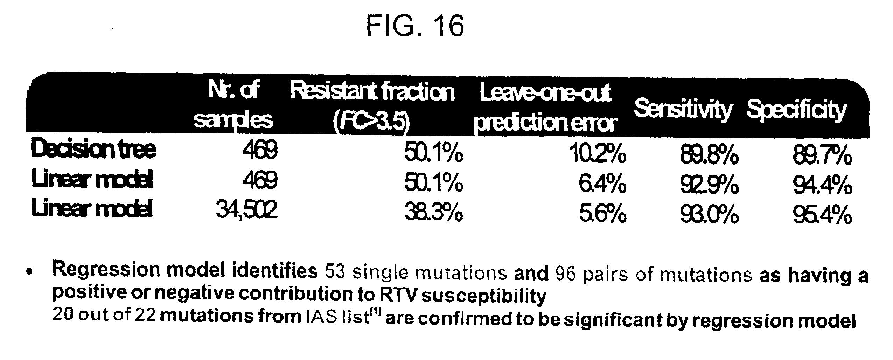

The predicted phenotype was compared to the measured phenotype in a leave-one-out cross-validation, demonstrating a root mean square error of 0.31 (logFR) (see Figure 15. The error rate of the linear modeling method [5.62% (sensitivity=93.0%, specificity = 95.4%)], compared favourably to a decision tree-based model [Beerenwinkel, PNAS 99, (2002) 8271-8276] [10.2% (sensitivity=89.8%, specificity=89.7%)] (see Figure 16).

The robustness of the algorithm as a function of the size of the input dataset was assessed using smaller subsets of data. Nine of 22 IAS resistance-associated mutations for RTV could be identified with subsets ≥ 5% (1600 isolates) of the original data. However, the accuracy of the predicted contribution of the mutations improved with increasing dataset sizes up to 50% of the original database (median standard error of the predicted contributions decreased 50%). Some secondary mutations (e.g. 10R, 32I, 82S) were identified as having a significant contribution to resistance only when the subset size reached a similar 50% level.

**Example 7: Comparison of genotype-to-phenotype prediction for different Artificial Intelligence techniques**

Analyses were performed on matching genotype/phenotype datasets for all 16 HIV inhibitors currently available. The matching genotype/phenotype datasets consisted of approximately 30,000 data points for most drugs except Lopinavir and Tenofovir.

As an example, the following results for Ritonavir were obtained:

● A log(*FR*) root mean squared error of 0.31 (MSE=0.096).

● A classification error of 5.6% (with regard to a standard cutoff applied by the inventors of *FR=3.5)*. (sensitivity=93.0%, specificity=95.4%).

● Regression model identified 53 single mutations and 96 pairs of mutations. 20 out of 22 mutations of the IAS list are confirmed in this model.

Table 28

| | $r^2$ | CV-MSE* (in log (FR)) | Classification error | Nr of samples | Coverage | Model inspection possible |
|---|---|---|---|---|---|---|
| Neural network *(Lopinavir)*[1,2] | 0.88 | n/a | n/a | 1,322 | 100% | N |
| Support Vector Machine *(Ritonavir)*[4] | 0.79 | 0.176 | n/a | 652 | 100% | N |
| Support Vector Machines *(Tibotec* data, *Ritonavir)* | 0.81 | 0.144 | n/a | 17,453 | 100% | N |
| Decision tree *(Ritonavir)*[3] | | *Classification only* | 10.2% | 469 | 100% | Y |
| Clustering *(Indinavir)*[5] | n/a | *n/a* | 15.3% | 1,152 | 100% | N |
| Self Organizing Map *(Saquinavir)*[2] | | *Classification only* | 15% | 811 (38 matching) | 84% | N |
| Linear regression modeling *(Virco* data, *Ritonavir)* | 0.88 | 0.096 | 5.6% | 34,502 | 100% | Y |

(*) CV-MSE: cross-validation mean squared error. Depending on the analysis, it is a leave-one-out or a 10-fold cross-validation [1] A 28-Mutation Neural Network Model that Accurately Predicts Phenotypic Resistance to Lopinavir (LPV) D Wang, R Harrigan and BA Larder, Antiviral Therapy 2001 (Supplement 1): 105 [2] *Predicting HIV Drug Resistance With Neural Networks* S Drághici, R Potter, Bioinformatics, Vol. 19 no. 1, 2003 (p. 98-107).

[3] *Geno2pheno: Interpreting Genotypic HIV Drug Resistance Tests* N Beerenwinkel et al., Intelligent Systems in Biology, Nov/Dec 2001.

[4] *Geno2pheno: estimating phenotypic drug resistance from HIV-1 genotypes* N Beerenwinkel et al., Nucleic Acids Research, 2003, Vol. 31, No 13.

[5] *Predicting phenotype from genotype: a comparison of statistical methods* A Foulkes et al.

Neural networks, support vector machine and clustering techniques are no suitable candidates for a quantitative prediction system with the requirement that such a system should have a descriptive power in order to be inspectable by experts and to be able to motivate certain predictions to customers. Decision trees allow experts to have insight in how the decision process works. But decision trees require massive amounts of data to build complex trees, since the information in the data is not optimally used.

Based on the performed analyses and the linear regression modelling feasibility study, linear regression modelling seems to meet the requirements for a quantitative prediction system. Linear regression seems to outperform other examined techniques and offers the possibility of inspection to HIV-experts. Considering the lower error rates, the linear regression modelling allows the optimisation of drug therapy in patients.

**Example 8: Comparison of the regression linear model with a rules-based algorithm**

The regression linear model developed by the inventors was used to test the drug-associated resistance of one HIV-1 sample on nevirapine, delavirdine, and efavirenz. In parallel, the same sample was run in a rules-based algorithm methodology as described in WO01/79540.

The results were expressed in FR, or fold change in IC50 or EC50, relative to reference wild-type virus, which is the drug concentration at which 50% of the enzyme activity is inhibited, and is expressed in μM units. The FR cutoffs values for normal susceptible ranges were 8, 10, and 6, for nevirapine, delavirdine, and efavirenz, respectively.

The phenotypic antiviral experiment was taken as the gold-standard by the inventors. Said phenotypic antiviral experiment was perfonned as described in WO97/27480. Results of the three methodologies are enclosed in Table 3 below.

According to the results obtained by the rules-based algorithm, the patient's sample would be susceptible to all three drugs. However, when the results obtained by the linear regression model were considered, the sample showed resistance against nevirapine and efavirenz and susceptibility against delavirdine. These last results were confirmed by the phenotypic antiviral experiments.

Table 29

| drug | Rules-based algorithm | | Linear regression model | Phenotypic antiviral experiment |
|---|---|---|---|---|
| | Matches in database | FR | FR | FR |
| Nevirapine | 321 | 3.6 | 176.1 | >89 |
| delavirdine | 301 | 1.9 | 3.5 | 3.7 |
| efavirenz | 300 | 1.9 | 12.9 | 17.5 |

## Claims

1. A computer-based method for quantitating the individual contribution of a mutation or combination of mutations to the drug resistance phenotype exhibited by HIV, said method comprising the step of performing a linear regression analysis using data from a dataset of matching genotypes and phenotypes,
   wherein the log fold resistance, pFR, of each HIV strain is modelled as the sum of all the individual resistance contributions for each of the mutations or combinations of mutations that occur in HIV according to the following equation;

$$pFR = \beta_A M_A + \beta_B M_B + \beta_n M_n + \cdots + \beta_Z M_Z + \varepsilon$$

   wherein each individual resistance contribution is calculated by multiplying a mutation factor, $M_A$, $M_B$, ..., $M_Z$, for each mutation or combination of mutations by a resistance coefficient $\beta_A$, $\beta_B$, .., $\beta_Z$;
   wherein for a combination of mutations, the mutation factor $M_n$ represents the co-occurrence of one mutation with other one or more mutations and the coefficient $\beta_n$ represents the synergy or antagonism between the one mutation with the other one or more mutations;
   wherein the mutation factor assigned to each mutation or combination of mutations reflects the degree to which that mutation or combination of mutations is present in the HIV strain and, if present, to which degree the mutation is present in a mixture;
   wherein each resistance coefficient reflects the contribution of the mutation or combination of mutations to the fold resistance exhibited by the strain;
   and wherein the error term $\varepsilon$, represents the difference between the modelled prediction and the experimentally determined measurement.

2. A method according to claim 1, wherein correlations are removed from the dataset for correlated mutations where not all correlated mutations contribute to the drug resistance phenotype, using an algorithm to track the change in pFR for each mutation as the effects of individual mutations or combinations of mutations are removed from the dataset.

3. A method according to claim 2, wherein the algorithm performs the following steps:

   a) calculate average pFR for all mutations with a sufficient count in the database to be significant;
   b) determine the extremes (maximum, minimum), and select the mutation with the pFR furthest away from the global average;
   c) remove all virus strains that have the selected mutation from the dataset and reiterate from step a);
   d) stop when the selected mutation in step b) has an average pFR that approximates to the global average;

such that removing virus strains with a certain resistance causing mutation results in an increase of the average pFR for correlating mutations, which thus have a higher average pFR.

4. A method according to any one of claims 1-3, wherein the algorithm performs the following steps:

   a) calculate correlation coefficient between all mutations with a sufficient count in the database and the pFR;
   b) determine the extremes (maximum, minimum), and select the mutation with the highest absolute value of correlation coefficient;
   c) calculate a linear model for the pFR with the selected mutation(s) (from step b), all previous iterations);
   d) take the residue;
   e) calculate correlation coefficient between all mutations with a sufficient count in the database and the residue;
   f) determine the extremes (maximum, minimum), and select the mutation with the highest absolute value of correlation coefficient;
   g) calculate a linear model for the pFR with the selected mutation(s) (from step f), all previous iterations); and
   h) reiterate from step d);
   i) stop when the selected mutation in step g) has a correlation coefficient that approximates to zero.

5. A method according to any one of the preceding claims, wherein censored values in the genotype / phenotype database are replaced by a maximum likelihood estimation.

6. A method according to claim 5, wherein for each iteration of the linear regression, the following steps are performed until the predictions converge:

   a) Calculate a linear regression model without censored values;
   b) Use the phenotypic measured value $V_0$ as if the censor was " = ", e.g. when a result is expressed as -log $FR$ < 4, we will treat $V_0$ as -log $FR$ =4;
   c) Look at the prediction P from the model and apply either:

   When case '<'-censor:

   ■ $P < V_0 - 0.798\ \sigma$ (center of gravity of half Gaussian distribution)

   o Remove value from training data for next iteration

   ■ $V_0 - 0.798\ \sigma \leq P < V_0$

   o Use $V' = V_0 - 0.798\ \sigma$ for next iteration

   ■ $V_0 \leq P$

   o Use V' centre of gravity of tail (<V) of a normal distribution N (P, $\sigma$) as value for next iteration.

   When case '>'-censor:

   ■ $P > V_0 + 0.798\ \sigma$ (center of gravity of half Gaussian distribution)

   o Remove value from training data for next iteration

   ■ $V_0 + 0.798\ \sigma \geq P > V_0$

   o Use $V' = V_0 - 0.798\ \sigma$ for next iteration

   ■ $V_0 \geq P$

   o Use V' centre of gravity of tail (>V) of a normal distribution N (P, $\sigma$) as value for next iteration.

   d) Calculate a linear regression model and for the censored values in the linear regression model, either remove the data-point from the training set, or use V' instead of the censored phenotypes measurement, as described in step c);
e) Re-iterate from step b) until prediction converges.

7. A method of identifying a mutation that effects the degree of drug resistance exhibited by an HIV strain using a method according to any one of the preceding claims.

8. A method according to claim 1 wherein the contribution of a mutation pattern to the drug resistance phenotype exhibited by an HIV strain is calculated, said method comprising the steps of:

a) obtaining a genetic sequence of said HIV strain,
b) identifying the pattern of mutations in said genetic sequence, wherein said mutations are associated with resistance or susceptibility to drug therapy, and
c) calculating the fold resistance of the HIV strain as compared to the wild type HIV strain by performing a linear regression analysis according to claim 1.

9. A method according to claim 8, which incorporates a method according to any one of claims 1-7.

10. A method according to any one of the preceding claims, wherein in the case of small datasets for particular mutations or combinations of mutations, the method is applied recursively to the set of virus strains that exhibit those particular mutations or combinations of mutations.

11. Use of a method according to any one of claims 1-6 for assessing the efficiency of a patient's therapy or for evaluating or optimizing a therapy.

12. A computer apparatus or computer-based system adapted to perform the method of any one of the claims 1-10.

13. A computer program product for use in conjunction with a computer, said computer program comprising a computer readable storage medium and a computer program mechanism embedded therein, the computer program mechanism comprising a module that is configured so that upon receiving a request to quantify the individual contribution of a mutation or combination of mutations to the drug resistance phenotype exhibited by HIV, or to calculate the quantitative contribution of a mutation pattern to the drug resistance phenotype exhibited by an HIV strain, it performs a method according to any one of claims 1-10.

**Patentansprüche**

1. Computerbasiertes Verfahren zur Quantifizierung des individuellen Beitrag einer Mutation oder einer Kombination von Mutationen zu dem Arzneimittelresistenz-Phänotyp, den HIV aufweist, wobei das Verfahren den Schritt umfasst, dass man eine lineare Regressionsanalyse unter Verwendung von Daten aus einem Datensatz übereinstimmender Genotypen und Phänotypen durchführt,
wobei log -fache Resistenz, *pFR*, jedes HIV-Stamms als Summe aller einzelnen Resistenz-Beiträge jeder der Mutationen oder Kombinationen von Mutationen, die in HIV auftreten, gemäß der folgenden Gleichung modelliert wird:

$$pFR = \beta_A M_A + \beta_B M_B + \beta_n M_n + ... + \beta_Z M_Z + \varepsilon \text{ ,}$$

wobei jeder einzelne Resistenz-Beitrag berechnet wird, indem man einen Mutationsfaktor, *$M_A$, $M_B$, ...., $M_Z$,* für jede Mutation oder Kombination von Mutationen mit einem Resistenzkoeffizienten, $\beta_A$, $\beta_B$, ..., $\beta_Z$, multipliziert,
wobei für eine Kombination von Mutationen der Mutationsfaktor *$M_n$* für das gleichzeitige Auftreten einer Mutation mit einer oder mehreren anderen Mutation(en) steht und der Koeffizient $\beta_n$ für die Synergie oder den Antagonismus zwischen der einen Mutation und der einen oder den mehreren anderen Mutation(en) steht, wobei der Mutationsfaktor, der jeder Mutation oder Kombination von Mutationen zugewiesen wird, den Grad widerspiegelt, in dem diese Mutation oder Kombination von Mutationen in dem HIV-Stamm vorliegt und, falls vorhanden, in welchem Grad die Mutation in einem Gemisch vorliegt,
wobei jeder Resistenzkoeffizient den Beitrag der Mutation oder Kombination von Mutationen zu der -fachen Resistenz widerspiegelt, die der Stamm aufweist,
und wobei der Fehlerterm $\varepsilon$ für die Differenz zwischen der modellierten Vorhersage und der experimentell bestimmten Messung steht.

2. Verfahren nach Anspruch 1, wobei Korrelationen aus dem Datensatz für korrelierte Mutationen entfernt werden, wenn nicht alle korrelierten Mutationen zu dem Resistenz-Phänotyp beitragen, wobei ein Algorithmus verwendet wird, mit dem die Veränderung in *pFR* für jede Mutation verfolgt werden, während die Wirkungen einzelner Mutationen oder Kombinationen von Mutationen aus dem Datensatz entfernt werden.

3. Verfahren nach Anspruch 2, wobei der Algorithmus die folgenden Schritte durchführt:

a) Berechnen des durchschnittlichen *pFR* für alle Mutationen mit einem ausreichenden Zählergebnis in der Datenbank, dass sie signifikant sind,
b) Bestimmen der Extremwerte (Maximum, Minimum), und Auswählen der Mutation mit dem *pFR*, der von dem globalen Durchschnitt am weitesten entfernt ist,
c) Entfernen aller Virusstämme, die die aus dem Datensatz ausgewählte Mutation aufweisen, und mehrmaliges Wiederholen von Schritt a),
d) Aufhören, wenn die in Schritt b) ausgewählte Mutation einen mittleren *pFR* aufweist, der sich dem globalen Durchschnittswert annähert,

so dass das Entfernen von Virusstämmen mit einer bestimmten Resistenz-verursachenden Mutation zu einer Zunahme des durchschnittlichen *pFR* für korrelierende Mutationen führt, die somit einen höheren durchschnittlichen *pFR* besitzen.

4. Verfahren nach einem der Ansprüche 1-3, wobei der Algorithmus die folgenden Schritte durchführt:

a) Berechnen des Korrelationskoeffizienten zwischen allen Mutationen mit einem ausreichenden Zählergebnis in der Datenbank und des *pFR*,
b) Bestimmen der Extremwerte (Maximum, Minimum), und Auswählen der Mutation mit dem höchsten Absolutwert des Korrelationskoeffizienten,
c) Berechnen eines linearen Modells für den pFR mit der/den ausgewählten Mutation(en) (aus Schritt b), alle vorherigen Iterationen),
d) Verwenden des Rests,
e) Berechnen des Korrelationskoeffizienten zwischen allen Mutationen mit einem ausreichenden Zählergebnis in der Datenbank und dem Rest,
f) Bestimmen der Extremwerte (Maximum, Minimum) und Auswählen der Mutation mit dem höchsten Absolutwert des Korrelationskoeffizienten,
g) Berechnen eines linearen Modells für den pFR mit der/den ausgewählten Mutation(en) (aus Schritt f), alle vorherigen Iterationen) und
h) mehrmaliges Wiederholen von Schritt d) an,
i) Aufhören, wenn die ausgewählte Mutation in Schritt g) einen Korrelationskoeffizienten aufweist, der sich Null annähert.

5. Verfahren nach einem der vorhergehenden Ansprüche, wobei zensierte Werte in der Genotyp/Phänotyp-Datenbank durch eine Schätzung der maximalen Wahrscheinlichkeit ersetzt werden.

6. Verfahren nach Anspruch 5, wobei für jede Iteration der linearen Regression die folgenden Schritte durchgeführt werden, bis die Vorhersagen konvergieren:

a) Berechnen eines linearen Regressionsmodells ohne zensierte Werte,
b) Verwenden des phänotypisch gemessenen Werts $V_0$, als sei der Censor "=", z. B. wenn ein Ergebnis als -log *FR* < 4 ausgedrückt wird, behandeln wir $V_0$ als -log *FR* = 4,
c) Ansehen der Vorhersage P aus dem Modell und Anwenden von entweder:

im Falle eines Censors "<":

■ P < $V_0$ - 0,798 σ (Schwerpunkt der Halb-Normalverteilung)

○ Entfernen des Werts aus den Trainingsdaten für die nächste Iteration

- $V_0 - 0{,}798\,\sigma \leq P < V_0$

  ○ Verwendung von V' = $V_0 - 0{,}798\,\sigma$ für die nächste Iteration

- $V_0 \leq P$

  ○ Verwendung von V' Schwerpunkt des Schwanzes (< V) einer Normalverteilung N(P, $\sigma$) als Wert für die nächste Iteration.

Im Falle eines Censors ">":

- $P > V_0 + 0{,}798\,\sigma$ (Schwerpunkt der Halb-Normalverteilung)

  ○ Entfernen des Werts aus den Trainingsdaten für die nächste Iteration

- $V_0 + 0{,}798\,\sigma \geq P > V_0$

  ○ Verwenden von V' = $V_0 - 0{,}798\,\sigma$ für die nächste Iteration

- $V_0 \geq P$

  ○ Verwenden von V' Schwerpunkt des Schwanzes (> V) einer Normalverteilung N(P, $\sigma$) als Wert für die nächste Iteration.

d) Berechnen eines linearen Regressionsmodells und, bezüglich der zensierten Werte in dem linearen Regressionsmodell, entweder Entfernen des Datenpunkts aus dem Trainingssatz oder Verwenden von V' anstelle der Messung der zensierten Phänotypen, wie im Schritt c) beschrieben,
e) mehrmaliges Wiederholen ab Schritt b), bis die Vorhersage konvergiert.

7. Verfahren zur Identifizierung einer Mutation, die den Grad der Arzneimittelresistenz, den ein HIV-Stamm aufweist, beeinflusst, unter Verwendung eines Verfahrens nach einem der vorhergehenden Ansprüche.

8. Verfahren nach Anspruch 1, wobei der Beitrag eines Mutationsmusters zu dem Arzneimittelresistenz-Phänotyp, den ein HIV-Stamm aufweist, berechnet wird, wobei das Verfahren die folgenden Schritte umfasst:

a) Erhalten einer genetischen Sequenz des HIV-Stamms,
b) Identifizieren des Musters von Mutationen in der genetischen Sequenz, wobei diese Mutationen mit einer Resistenz gegen oder einer Empfänglichkeit für eine Arzneimitteltherapie einhergehen, und
c) Berechnen der -fachen Resistenz des HIV-Stamms im Vergleich zum Wildtyp-HIV-Stamm, indem man eine lineare Regressionsanalyse nach Anspruch 1 durchführt.

9. Verfahren nach Anspruch 8, das ein Verfahren nach einem der Ansprüche 1-7 umfasst.

10. Verfahren nach einem der vorhergehenden Ansprüche, wobei im Falle kleiner Datensätze für bestimmte Mutationen oder Kombinationen von Mutationen das Verfahren rekursiv auf den Satz von Virusstämmen, die diese bestimmten Mutationen oder Kombinationen von Mutationen aufweisen, angewendet wird.

11. Verwendung eines Verfahrens nach einem der Ansprüche 1-6 zur Untersuchung der Effizienz der Therapie eines Patienten oder zur Bewertung oder Optimierung einer Therapie.

12. Computervorrichtung oder computerbasiertes System, das für die Durchführung des Verfahrens nach einem der Ansprüche 1-10 ausgelegt ist.

13. Computerprogrammprodukt, das in Verbindung mit einem Computer verwendet wird, wobei das Computerprogramm ein computerlesbares Speichermedium und einen darin eingebetteten Computerprogramm-Mechanismus umfasst, wobei der Computerprogramm-Mechanismus ein Modul umfasst, das derart konfiguriert ist, dass es bei Empfang einer Anfrage nach der Quantifizierung des individuellen Beitrags einer Mutation oder einer Kombination von Mutationen zu dem Arzneimittelresistenz-Phänotyp, den HIV aufweist, oder nach der Berechnung des quantitativen Beitrags eines Mutationsmusters zu dem Arzneimittelresistenz-Phänotyp, den HIV aufweist, ein Verfahren nach einem der Ansprüche 1-10 durchführt.

**Revendications**

1. Procédé informatique pour quantifier la contribution individuelle d'une mutation ou combinaison de mutations au phénotype de pharmacorésistance présenté par le VIH, ledit procédé comprenant l'étape d'exécution d'une analyse de régression linéaire en utilisant des données d'un ensemble de données de génotypes et phénotypes correspondants,

   le facteur de résistance logarithmique, pFR, de chaque souche de VIH étant modélisé comme étant la somme de l'ensemble des contributions individuelles à la résistance pour chacune des mutations ou combinaison de mutations qui surviennent dans le VIH selon l'équation suivante ;

$$pFR = \beta_A M_A + \beta_B M_B + \beta_n M_n + \ldots + \beta_Z M_Z + \varepsilon$$

   chaque contribution individuelle à la résistance étant calculée en multipliant un facteur de mutation, $M_A$, $M_B$, ..., $M_Z$, pour chaque mutation ou combinaison de mutations par un coefficient de résistance $\beta_A$, $\beta_B$, ..., $\beta_Z$;

   pour une combinaison de mutations, le facteur de mutation $M_n$ représente la co-occurrence d'une mutation avec une ou plusieurs mutations et le coefficient $\beta_n$ représente la synergie ou l'antagonisme entre la mutation et les autres une ou plusieurs mutations ;

   le facteur de mutation attribué à chaque mutation ou combinaison de mutations reflétant le degré auquel cette mutation ou combinaison de mutations est présent dans la souche de VIH et, le cas échéant, à quel degré la mutation est présente dans un mélange ;

   chaque coefficient de résistance reflétant la contribution de la mutation ou combinaison de mutations au facteur de résistance présenté par la souche ;

   et le terme d'erreur $\varepsilon$, représente la différence entre la prédiction modélisée et la mesure déterminée de façon expérimentale.

2. Procédé selon la revendication 1, dans lequel les corrélations sont enlevées de l'ensemble de données pour des mutations corrélées où les mutations corrélées ne contribuent pas toutes au phénotype de pharmacorésistance, en utilisant un algorithme pour suivre la modification de pFR pour chaque mutation au fur et à mesure que les effets de mutations individuelles ou combinaisons de mutations sont enlevés de l'ensemble de données.

3. Procédé selon la revendication 2, dans lequel l'algorithme exécute les étapes suivantes :

   a) calculer le pFR moyen pour toutes les mutations en nombre suffisant dans la base de données pour être significatives ;
   b) déterminer les extrêmes (maximum, minimum), et sélectionner la mutation avec le pFR le plus éloigné de la moyenne globale ;
   c) éliminer toutes les souches de virus qui ont la mutation sélectionnée de l'ensemble de données et réitérer à partir de l'étape a) ;
   d) arrêter lorsque la mutation sélectionnée dans l'étape b) a un pFR moyen qui est proche de la moyenne globale ;

   de sorte que l'élimination des souches de virus ayant une certaine résistance causant des mutations conduit à une augmentation du pFR moyen pour corréler des mutations, qui ont donc un pFR moyen plus élevé.

4. Procédé selon l'une quelconque des revendications 1 à 3, dans lequel l'algorithme exécute les étapes suivantes :

   a) calculer le coefficient de corrélation entre toutes les mutations ayant un nombre suffisant dans la base de données et le pFR ;
   b) déterminer les extrêmes (maximum, minimum), et sélectionner la mutation avec la valeur absolue la plus élevée de coefficient de corrélation ;
   c) calculer un modèle linéaire pour le pFR avec la/les mutation(s) sélectionnée(s) (de l'étape b), toutes les itérations précédentes) ;
   d) prélever le résidu ;

e) calculer le coefficient de corrélation entre toutes les mutations avec un nombre suffisant dans la base de données et le résidu ;

f) déterminer les extrêmes (maximum, minimum) et sélectionner la mutation avec la valeur absolue la plus élevée de coefficient de corrélation ;

g) calculer un modèle linéaire pour le pFR avec la/les mutation(s) sélectionnée(s) (de l'étape f), toutes les itérations précédentes) ; et

h) réitérer à partir de l'étape d) ;

i) arrêter lorsque la mutation sélectionnée dans l'étape g) a un coefficient de corrélation qui est proche de zéro.

5. Procédé selon l'une quelconque des revendications précédentes, dans lequel les valeurs censurées dans la base de données de génotype /phénotype sont remplacées par une estimation de probabilité maximale.

6. Procédé selon la revendication 5, dans lequel, pour chaque itération de la régression linéaire, les étapes suivantes sont effectuées jusqu'à ce que les prédictions convergent :

a) calculer un modèle de régression linéaire sans valeurs censurées ;

b) utiliser la valeur mesurée phénotypique $V_0$ comme si le censeur était « = », par exemple, lorsqu'un résultat est exprimé sous la forme -logFR < 4, nous traitons $V_0$ comme étant -logFR = 4 ;

c) examiner la prédiction P du modèle et appliquer soif :

lorsque le cas « < »-censeur :

- $P < V_0 -0{,}798\,\sigma$ (centre de gravité de distribution semi-gaussienne)

- éliminer la valeur des données d'apprentissage pour l'itération suivante

- $V_0 -0{,}798\,\sigma \leq P < V_0$

- utiliser $V' = V_0 -0{,}798\,\sigma$ pour l'itération suivante

- $V_0 \leq P$

- utiliser le centre de gravité V' de la queue (<V) d'une distribution normale $N(P,\sigma)$ en tant que valeur pour l'itération suivante,

lorsque le cas « > »-censeur :

- $P > V_0 +0{,}798\,\sigma$ (centre de gravité de distribution semi-gaussienne)

- éliminer la valeur des données d'apprentissage pour l'itération suivante

- $V_0 +0{,}798\,\sigma \geq P > V_0$

- utiliser $V' = V_0 -0{,}798\,\sigma$ pour l'itération suivante

- $V_0 \geq P$

- utiliser le centre de gravité V' de la queue (>V) d'une distribution normale $N(P,\sigma)$ en tant que valeur pour l'itération suivante,

d) calculer un modèle de régression linéaire et pour les valeurs censurées dans le modèle de régression linéaire, enlever le point de données de l'ensemble d'apprentissage, ou utiliser V' au lieu de la mesure de phénotypes censurés, comme décrit dans l'étape c) ;

e) réitérer à partir de l'étape b) jusqu'à ce que la prédiction converge.

7. Procédé d'identification d'une mutation qui affecte la pharmacorésistance présentée par une souche VIH en utilisant un procédé selon l'une quelconque des revendications précédentes.

**8.** Procédé selon la revendication 1 dans lequel la contribution d'un profil de mutation au phénotype de pharmacorésistance présenté par une souche de VIH est calculée, ledit procédé comprenant les étapes consistant à :

a) obtenir une séquence génétique de ladite souche de VIH,
b) identifier le motif de mutations dans ladite séquence génétique, lesdites mutations étant associées à une résistance ou une sensibilité à une pharmacothérapie, et
c) calculer le facteur de résistance de la souche de VIH comparée à la souche de VIH de type sauvage en effectuant une analyse de régression linéaire selon la revendication 1.

**9.** Procédé selon la revendication 8, qui incorpore un procédé selon l'une quelconque des revendications précédentes 1 à 7.

**10.** Procédé selon l'une quelconque des revendications précédentes, dans lequel dans le cas de petits ensembles de données pour des mutations ou combinaisons de mutations particulières, le procédé est appliqué de façon récursive à l'ensemble de souches de virus qui présente ces mutations ou combinaisons de mutations particulières.

**11.** Utilisation d'un procédé selon l'une quelconque des revendications 1 à 6 pour évaluer l'efficacité d'une thérapie d'un patient ou pour évaluer ou optimiser une thérapie.

**12.** Appareil informatique ou système informatique adapté pour exécuter le procédé de l'une quelconque des revendications 1 à 10.

**13.** Procédé informatique pour utilisation conjointement avec un ordinateur, ledit programme informatique comprenant un support de stockage lisible par ordinateur et un mécanisme de programme informatique incorporé dans celui-ci, le mécanisme de programme informatique comprenant un module qui est configuré de sorte qu'après la réception d'une demande de quantifier la contribution individuelle d'une mutation ou combinaison de mutations au phénotype de pharmacorésistance présenté par le VIH, ou de calculer la contribution quantitative d'un profil de mutation au phénotype de pharmacorésistance présenté par une souche de VIH, il exécute un procédé selon l'une quelconque des revendications 1 à 10.

FIG. 1

Drug resistance model

Large geno/pheno database

Drug resistance model

Single patient analysis

Patient blood sample

Genotyping

Analysis

Predicted phenotype

In vitro phenotyping

Measured phenotype

FIG. 2

## FIG. 3

EP 1 636 727 B1

pFR

FIG. 4

pFR

EP 1 636 727 B1

FIG. 5

pFR

## FIG. 6

**71 I** (291 virus strains)

pFR

**71 I& 48V & 84V**
(182 virus strains)

pFR

EP 1 636 727 B1

FIG. 7

## FIG. 8

EP 1 636 727 B1

# FIG. 9

EP 1 636 727 B1

| Sample | Position | | | | | | | | | | | | | | | | | | | | | | | |
|--------|---|----|----|----|----|----|----|----|----|----|----|----|----|----|----|----|----|----|----|----|----|----|----|----|
| | 3 | 10 | 12 | 15 | 19 | 20 | 24 | 30 | 32 | 36 | 37 | 41 | 46 | 48 | 54 | 62 | 63 | 71 | 74 | 75 | 77 | 82 | 84 | 93 |
| V1 | I | | | | | | | | I/V | N | | I | | | | | P | | | | | A | | L |
| V2 | I | I | | V | R | | | | | I | N | K | | V | T | | T | V | A | | | A | | L |
| V3 | I | | S | | I | | | N | | | N | | | | | V | P | | | I | I | | | L |
| V4 | I | | | | | | I | | | | N | | I/M | | I/V | P | V | | | | | | T | I/V |

# FIG. 10

| Regression model | | Virus sample | | | |
|---|---|---|---|---|---|
| Mutation | log(FC) shift | V1 | V2 | V3 | V4 |
| 24I | 0.50 | | | | 1 |
| 30N | 0.39 | | | 1 | |
| 32I | 0.32 | ½ | | | |
| 46I | 0.21 | 1 | | | ½ |
| 54T | 1.33 | | 1 | | |
| 54V | 0.52 | | | | ½ |
| 82A | 0.63 | 1 | 1 | | |
| 82T | 0.59 | | | | 1 |
| 84V | 0.67 | | | | ½ |
| Other interactions | | -0.14 | 0.22 | -0.17 | -0.02 |
| predicted log(FC) | | 0.86 | 2.18 | -0.56 | 1.77 |
| measured log(FC) | | 0.89 | 2.19 | -0.50 | 1.81 |

EP 1 636 727 B1

FIG. 11

# FIG. 12

$P < V$ - $0.798\,\sigma$ $\qquad$ $V$ - $0.798\,\sigma < P < V$ $\qquad$ $V < P$

EP 1 636 727 B1

## FIG. 13

| Mutation | 1<sup>st</sup> order log($FC$) shift | Prevalence in dataset |
|---|---|---|
| 10I* | 2<sup>nd</sup> order terms only | 9,707 |
| 10R | 0.35 | 106 |
| 10V* | 0.15 | 1,269 |
| 20M | 2<sup>nd</sup> order terms only | 436 |
| 20R* | 2<sup>nd</sup> order terms only | 2,093 |
| 32I | 0.32 | 845 |
| 33F* | 2<sup>nd</sup> order terms only | 1,074 |
| 36I* | 2<sup>nd</sup> order terms only | 8,473 |
| 46I* | 0.21 | 4,115 |
| 46L | 0.19 | 1,745 |
| 54L | 0.33 | 367 |
| 54V* | 0.52 | 4,553 |
| 71T | 2<sup>nd</sup> order terms only | 2,611 |
| 71V | 2<sup>nd</sup> order terms only | 7,261 |
| 82A* | 0.63 | 4,886 |
| 82F | 0.92 | 290 |
| 82T* | 0.59 | 642 |
| 82S | 1.17 | 120 |
| 84V* | 0.67 | 3,558 |
| 90M* | 0.38 | 9,609 |

EP 1 636 727 B1

## FIG. 14

| Mutation | log(FC) shift | Prevalence in dataset |
|---|---|---|
| 24I | 0.50 | 1,027 |
| 30N | -0.39 | 1,715 |
| 54T | 1.33 | 155 |
| 73C | 0.45 | 357 |
| 73S | 0.38 | 2,224 |
| 73T | 0.53 | 559 |
| 82M | 0.66 | 33 |
| 84A | 1.73 | 70 |
| 84C | 0.79 | 67 |

EP 1 636 727 B1

# FIG. 15

EP 1 636 727 B1

## FIG. 16

| | Nr. of samples | Resistant fraction ($FC>3.5$) | Leave-one-out prediction error | Sensitivity | Specificity |
|---|---|---|---|---|---|
| Decision tree | 469 | 50.1% | 10.2% | 89.8% | 89.7% |
| Linear model | 469 | 50.1% | 6.4% | 92.9% | 94.4% |
| Linear model | 34,502 | 38.3% | 5.6% | 93.0% | 95.4% |

- Regression model identifies 53 single mutations and 96 pairs of mutations as having a positive or negative contribution to RTV susceptibility
  20 out of 22 mutations from IAS list[1] are confirmed to be significant by regression model

FIG. 17

FIG. 18

FIG. 19

Histogram (2)

Residue Binned

FIG. 20

Histogram

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0179540 A

- WO 9727480 A

### Non-patent literature cited in the description

- **LARDER et al.** *Science,* 1989, vol. 246, 1155-8
- **BEERENWINKEL et al.** *PNAS,* June 2002, vol. 99 (12), 8271-8276
- **SCHMIDT et al.** *AIDS,* August 2000, vol. 14 (12), 1731-1738
- **SEVIN et al.** *Journal of Infectious Diseases,* July 2000, vol. 182 (1), 59-67
- **SCHMIDT et al.** *AIDS Reviews,* vol. 4 (3), 148-156
- **MEISEL et al.** *Therapeutic Drug Monitoring,* February 2001, vol. 23 (1), 9-14
- **MEISEL et al.** *Pharmacogenetics,* 1997, vol. 7 (3), 241-246
- **SANGER F. ; NICHER. ; COULSON A.** *Proc. Nat. Acad. Sci.,* 1977, vol. 74, 5463-5467
- **GRABER J ; SMITH C. ; CANTOR C.** *Genet. Anal.,* 1999, vol. 14, 215-219

- **FODOR S P ; RAVA R P ; HUANG XC ; PEASE A C ; HOLMES C P ; ADAMS C L.** *Nature,* 1993, vol. 364, 555-6
- **SMITH ; WATERMAN.** *J Mol Biol,* 1981, vol. 147, 195-197
- **ALTSCHUL et al.** *J Mol Biol.,* 1990, vol. 215 (3), 403-10
- **PEARSON ; LIPMAN.** *Proc Natl Acad Sci USA,* 1988, vol. 85 (8), 2444-8
- **ALTSCHUL et al.** *Nucleic Acids Res.,* 1977, vol. 25 (17), 3389-402
- **THOMPSON et al.** *NAR,* 1994, vol. 22 (22), 4673-4680
- Bioinformatics: A practical guide to the analysis of genes and proteins. John Wiley and Sons, 1998
- **BEERENWINKEL.** *PNAS,* 2002, vol. 99, 8271-8276